(19) Europäisches Patentamt / European Patent Office / Office européen des brevets

(11) EP 4 338 593 A1

(12) EUROPEAN PATENT APPLICATION
published in accordance with Art. 153(4) EPC

(43) Date of publication:
**20.03.2024 Bulletin 2024/12**

(21) Application number: **22807426.6**

(22) Date of filing: **09.05.2022**

(51) International Patent Classification (IPC):
***A01N 43/836*** (2006.01) ***A01N 37/34*** (2006.01)
***A01N 37/50*** (2006.01) ***A01N 43/40*** (2006.01)
***A01N 43/42*** (2006.01) ***A01N 43/50*** (2006.01)
***A01N 43/54*** (2006.01) ***A01N 43/56*** (2006.01)
***A01N 43/653*** (2006.01) ***A01N 47/14*** (2006.01)
***A01N 47/24*** (2006.01) ***A01N 55/02*** (2006.01)
***A01P 3/00*** (2006.01) ***C07D 413/10*** (2006.01)
***C07D 413/14*** (2006.01)

(52) Cooperative Patent Classification (CPC):
**A01N 37/34; A01N 37/50; A01N 43/40; A01N 43/42; A01N 43/50; A01N 43/54; A01N 43/56; A01N 43/653; A01N 43/82; A01N 47/14; A01N 47/24; A01N 55/02; A01P 3/00; C07D 413/10; C07D 413/14**

(86) International application number:
**PCT/JP2022/019639**

(87) International publication number:
**WO 2022/239725 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.05.2021 JP 2021080430**

(71) Applicant: **Nippon Soda Co., Ltd.**
**Tokyo 100-7010 (JP)**

(72) Inventors:
- **KUROYANAGI, Teruhiko**
**Odawara-shi, Kanagawa 250-0280 (JP)**
- **NAKAMURA, Yuka**
**Odawara-shi, Kanagawa 250-0280 (JP)**
- **SAIGA, Tomoyuki**
**Odawara-shi, Kanagawa 250-0280 (JP)**

(74) Representative: **Sonnenhauser, Thomas Martin**
**Wuesthoff & Wuesthoff**
**Patentanwälte PartG mbB**
**Schweigerstraße 2**
**81541 München (DE)**

(54) **AGRICULTURAL AND HORTICULTURAL FUNGICIDAL COMPOSITION**

(57) The present invention addresses the problem of providing an agricultural/horticultural fungicidal composition with excellent fungicidal activity and without any concern about chemical damage to useful plants. The present invention provides an agricultural/horticultural fungicidal composition comprising component (I) and component (II) as active ingredients, wherein the component (I) is a compound of formula (I) and the component (II) is, for example, a microbicide other than the component (I).

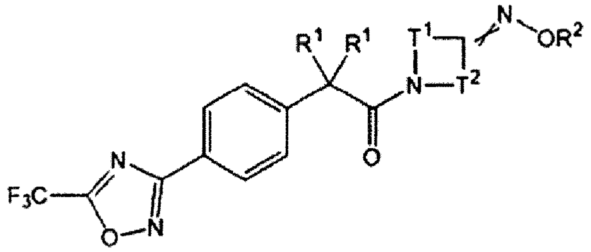

(I)



Processed by Luminess, 75001 PARIS (FR)

## Description

### Technical Field

The present invention relates to an agricultural/horticultural fungicidal composition. The present invention relates more specifically to an agricultural/horticultural fungicidal composition with excellent fungicidal activity and superior safety. The present application claims the priority of Japanese Patent Application No. 2021-080430, filed on May 11, 2021, the content of which is herein incorporated by reference.

### Background Art

For the cultivation of agricultural or horticultural crops, various compounds have been proposed that have disease control activity against crop diseases. To practically implement such compounds as agricultural/horticultural fungicides, the following is required including not only sufficiently high efficacy, but also hardly occurring drug resistance, occurrence of neither chemical damage to plants nor soil contamination, and low toxicity to livestock, fish, and other organisms.

WO 2019/022061 A describes an oxadiazol compound and an oxadiazol compound-containing fungicide for agricultural and horticultural use.

### Prior Art Documents

### Patent Documents

Patent Document 1 : WO 2019/022061 A

### Summary of the Invention

### Object to be Solved by the Invention

The present invention addresses the problem of providing an agricultural/horticultural fungicidal composition with better fungicidal activity and without any concern about chemical damage to useful plants.

### Means to Solve the Object

As a result of intensive research to solve the above problem, the present invention, which encompasses the following items, has been completed.

Specifically, the present invention is as follows.

[1] An agricultural/horticultural fungicidal composition comprising component (I) and component (II) as active ingredients, wherein the component (I) is at least one compound selected from a compound of the formula (I):

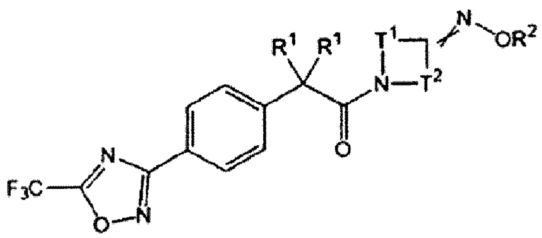

(I)

and a salt thereof, wherein

$R^1$ each independently represents a hydrogen atom, a $C_{1-6}$ alkyl group, or a $C_{1-6}$ alkoxy group;
$R^2$ represents a $C_{1-6}$ alkyl group, a $C_{1-6}$ alkyl group having at least one $G^1$, a $C_{2-6}$ chained unsaturated hydrocarbon group, a $C_{2-6}$ chained unsaturated hydrocarbon group having at least one $G^1$, a $C_{3-6}$ cycloalkyl group, a $C_{3-6}$ cycloalkyl group having at least one $G^2$, a $C_{6-10}$ aryl group, a $C_{6-10}$ aryl group having at least one $G^2$, a 5- to 6-membered heterocyclyl group, or a 5- to 6-membered heterocyclyl group having at least one $G^2$;
$G^1$ each independently represents a halogeno group, a hydroxyl group, a $C_{1-6}$ alkoxy group, a $C_{1-6}$ haloalkoxy

group, a $C_{1-6}$ alkylthio group, a $C_{1-6}$ alkylsulfinyl group, a $C_{1-6}$ alkylsulfonyl group, a $C_{3-6}$ cycloalkyl group, a $C_{6-10}$ aryl group, a $C_{6-10}$ aryl group having at least one $g^1$, a 5- to 6-membered heterocyclyl group, a 5- to 6-membered heterocyclyl group having at least one $g^1$, or a cyano group;
$G^2$ each independently represents a halogeno group, a $C_{1-6}$ alkyl group, a $C_{1-6}$ haloalkyl group, a hydroxyl group, a $C_{1-6}$ alkoxy group, a $C_{1-6}$ alkylthio group, a $C_{1-6}$ alkylsulfinyl group, a $C_{1-6}$ alkylsulfonyl group, a $C_{3-6}$ cycloalkyl group, a $C_{6-10}$ aryl group, a $C_{6-10}$ aryl group having at least one $g^1$, a 5- to 6-membered heterocyclyl group, a 5- to 6-membered heterocyclyl group having at least one $g^1$, a nitro group, or a cyano group;
$g^1$ each independently represents a halogeno group, a $C_{1-6}$ alkyl group, a $C_{1-6}$ haloalkyl group, a $C_{1-6}$ alkoxy group, a $C_{1-6}$ haloalkoxy group, a nitro group, or a cyano group;
$T^1$ represents a $C_{1-2}$ alkylene group or a $C_{1-6}$ alkylene group having at least one $X^4$;
$T^2$ represents a $C_{1-2}$ alkylene group or a $C_{1-6}$ alkylene group having at least one $X^4$; and
$X^4$ each independently represents a halogeno group or a $C_{1-6}$ alkyl group, and

wherein the component (II) is a fungicide other than the component (I).
[2] The agricultural/horticultural fungicidal composition according to [1], wherein the component (II) is at least one fungicide selected from the group consisting of the following:

(A) Agents acting on nucleic acid synthesis and metabolism:

A1) RNA polymerase I inhibitors
benalaxyl, benalaxyl-M, furalaxyl, metalaxyl, metalaxyl-M, oxadixyl, ofurace,
A2) adenosine deaminase inhibitors
bupirimate, dimethirimol, ethirimol,
A3) DNA/RNA biosynthesis inhibitors
hymexazole, octhilinone,
A4) DNA topoisomerase type II inhibitors
oxolinic acid,

(B) Agents acting on cytoskeleton and motor proteins:

B1) to B3) β-tubulin polymerization inhibitors
benomyl, carbendazim, fuberidazole, thiabendazole, thiophanate, thiophanate-methyl, diethofencarb, zoxamide, ethaboxam, chlorfenazole, debacarb, trichlamide, zarilamid,
B4) mitosis (point of action unknown) inhibitors
pencycuron,
B5) delocalization inhibitors of spectrin-like proteins
fluopicolide, fluopimomide,
B6) actin/myosin/fimbrin function inhibitors
phenamacril, metrafenone, pyriofenone,

(C) agents acting on respiration:

C1) complex I:NADH oxidoreductase inhibitors
diflumetorim, tolfenpyrad, fenazaquin,
C2) complex II:succinate dehydrogenase inhibitors
benodanil, flutolanil, mepronil, isofetamid, fluopyram, cyclobutrifluram, fenfuram, carboxin, oxycarboxin, thifluzamide, benzovindiflupyr, bixafen, fluindapyr, fluxapyroxad, furametpyr, inpyrfluxam, isopyrazam, penflufen, penthiopyrad, sedaxane, isoflucypram, pydiflumetofen, boscalid, pyraziflumid, flubeneteram, furmecyclox,
C3) complex III:cytochrome bc1 (ubiquinol oxidase) Qo site (cyt b gene) inhibitors
azoxystrobin, coumoxystrobin, enoxastrobin, flufenoxystrobin, picoxystrobin, pyraoxystrobin, mandestrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, methyltetraprole,
C4) complex III:cytochrome bc1 (ubiquinone reductase) Qi site inhibitors
cyazofamid, amisulbrom, fenpicoxamid, florylpicoxamid, metarylpicoxamid,
C5) deconjugation inhibitors of oxidative phosphorylation binapacryl, dinocap, meptyldinocap, fluazinam,
C6) inhibitors of oxidative phosphorylation and ATP synthase fentin acetate, fentin chloride, fentin hydroxide,

C7) ATP transport inhibitors
silthiofam,
C8) complex III:cytochrome bc1 (ubiquinone reductase) Qo site, stigmatellin-binding subsite inhibitors
ametoctradin,

(D) agents acting on amino acid and protein synthesis:

D1) methionine biosynthesis (cgs gene) inhibitors
cyprodinil, mepanipyrim, pyrimethanil,
D2) protein synthesis (ribosomal translation termination step) inhibitors
blasticidin S
D3), D4) protein synthesis (ribosomal translation initiation step) inhibitors
kasugamycin, kasugamycin hydrochloride, streptomycin,
D5) protein synthesis (ribosomal polypeptide elongation step) inhibitors
oxytetracycline

(E) agents acting on signal transduction:

E1) signal transduction (mechanism of action unknown) inhibitors
quinoxyfen, proquinazid,
E2) MAP/histidine kinase (os-2, HOG1) inhibitors in osmotic signal transduction
fenpiclonil, fludioxonil,
E3) MAP/histidine kinase (os-1, Daf1) inhibitors in osmotic signal transduction
clozolinate, dimethachlone, iprodione, procymidone, vinclozolin,

(F) agents acting on lipid biosynthesis or transport/cell membrane structure or function:

F1) dicarboxyimide-based fungicides
F2) phospholipid biosynthesis, methyltransferase inhibitors edifenphos, iprobenfos, pyrazophos, isoprothiolane,
F3) cell peroxidation inhibitors
biphenyl, chloroneb, dicloran, quintozene, tecnazene, tolclofos-methyl, etridiazole,
F4) cell membrane permeability, fatty acid inhibitors
iodocarb, propamocarb, propamocarb hydrochloride, prothiocarb,
F5) carboxylic acid amide (CAA)-based fungicides
F8) ergosterol binding inhibitors
natamycin,
F9) lipid homeostasis and transport/storage inhibitors
oxathiapiprolin, fluoxapiprolin,

(G) inhibitors of sterol biosynthesis in cell membrane

G1) demethylase (erg11/cyp51) inhibitors at C14 position of sterol biosynthesis
triforine, pyrifenox, pyrisoxazole, fenarimol, nuarimol, imazalil, oxpoconazole, pefurazoate, prochloraz, triflumizole, azaconazole, bitertanol, bromuconazole, cyproconazole, difenoconazole, diniconazole, epoxiconazole, etaconazole, fenbuconazole, fluquinconazole, flusilazole, flutriafol, hexaconazole, imibenconazole, ipconazole, mefentrifluconazole, metconazole, myclobutanil, penconazole, propiconazole, simeconazole, tebuconazole, tetraconazole, triadimefon, triadimenol, triticonazole, prothioconazole, fluoxytioconazole, furconazole, furconazole-cis, diniconazole-M,
G2) $\Delta$14-reductase and $\Delta 8 \rightarrow \Delta 7$-isomerase (erg24, erg2) inhibitors in sterol biosynthesis
aldimorph, dodemorph, dodemorph acetate, fenpropimorph, tridemorph, fenpropidin, piperalin, spiroxamine, buthiobate,
G3) 3-keto reductase (erg27) inhibitors in C4-position demethylation of sterol biosynthetic system
fenhexamid, fenpyrazamine,
G4) squalene epoxidase (erg1) inhibitors of sterol biosynthesis system
pyributicarb, naftifine, terbinafine,

(H) cell wall biosynthesis inhibitors:

H4) chitin synthase inhibitors
polyoxin, polyoxorim,
H5) cellulose synthase inhibitors
dimethomorph, flumorph, pyrimorph, benthiavalicarb, iprovalicarb, valifenalate, mandipropamid,

(I) cell wall melanin synthesis inhibitors:

I1) reductase inhibitors in melanin biosynthesis
fthalide, pyroquilon, tricyclazole,
I2) dehydratase inhibitors in melanin biosynthesis
carpropamid, diclocymet, fenoxanil,
I3) polyketide synthase inhibitors in melanin biosynthesis tolprocarb,

(P) agents acting to induce resistance in host plants:

P01 to P03) agents involving salicylic acid signal transduction
acibenzolar-S-methyl, probenazole, tiadinil, isotianil,
P04) polysaccharide elicitors
laminarin,
P05) anthraquinone elicitors
extract from *Reynoutria sachalinensis,*
P06) microbial elicitors
cell walls of *Bacillus mycoides* isolate J, *Saccharomyces cerevisiae* strain LAS117,
P07) phosphonates
fosetyl-Al or phosphorous acids and salts thereof (including, potassium phosphite, calcium phosphite, aluminum phosphite, sodium phosphite),
P08) agents involving salicylic acid signal transduction dichlobentiazox,

(U) agents with unknown mechanism of action:
cymoxanil, teclofthalam, triazoxide, flusulfamide, diclomezine, cyflufenamid, dodine, dodine free base, flutianil, ferimzone, tebufloquin, picarbutrazox, validamycin, bethoxazin, cyprofuram, flumetover, nitrothal-isopropyl, propamidine, ipflufenoquin, pyridachlometyl, pyrapropoyne, aminopyrifen, ipfentrifluconazole, quinofumelin, dipymetitrone, chloroinconazide, seboctylamine, flumetylsulforim, flufenoxadiazam,
(M) agents with multiple action point contact activity:

copper (different salts), basic copper sulfate, Bordeaux mixture, copper hydroxide, copper naphthenate, copper oxychloride, copper sulfate, cuprous oxide, oxine-copper, sulfur, lime sulfur, amobam, ferbam, mancozeb, maneb, metiram, propineb, thiram, zinc thiazole, zineb, ziram, captan, captafol, folpet, chlorothalonil, dichlofluanid, tolylfluanid; guazatine, guazatine acetates, iminoctadine, iminoctadine triacetate, iminoctadine trialbesilate, anilazine, dithianon,
quinomethionate, fluoroimide, methasulfocarb, dazomet, cufraneb, mancopper, polycarbamate,

(BM) biological pesticides/biological-derived pesticides with multiple mechanisms of action

a) plant extracts:
polypeptides (lectin), phenols, sesquiterpenes, triterpenoids, coumarins, terpene hydrocarbons, terpene alcohols, terpene phenols, extract from the cotyledons of lupine plantlets, extract from *Swinglea glutinosa,* extract from *Melaleuca alternifolia* (tea tree oil), plant oils (mixtures), eugenol, geraniol, thymol, alpha-pinene, alpha-terpinene, alpha-terpinol, alpha-terpinoline, gamma-terpinene, d-limonene, orange oil, linalool, menthol, ursolic acid, oleanolic acid, neem oil,
b) microorganisms (microbial strains or their extracts or metabolites):

fungi of the genus *Trichoderma,* including *Trichoderma atroviride, Trichoderma asperellum, Trichoderma harzianum,* or *Trichoderma virens,*
fungi of the genus *Gliocladium,* including *Gliocladium catenulatum,*
fungi of the genus *Clonostachys,* including *Cronostachys rosea,*
fungi of the genus *Coniothyrium,* including *Coniotylium minutans,*
fungi of the genus *Talaromyces,* incluidng *Talaromyces flavus,* yeast of the genus *Saccharomyces,*

including *Saccharomyces cerevisiae,*
bacteria of the genus *Bacillus,* including *Bacillus amyloliquefaciens, Bacillus subtilis, Bacillus simplex,*
bacteria of the genus *Paenibacillus,*
bacteria of the genus *Burkholderia,*
fungi of the genus *Fusarium,*
bacteria of the genus *Pseudomonas,* including *Pseudomonas chlororaphis, Pseudomonas fluorescens, Pseudomonas rhodesiae,*
bacteria of the genus *Streptomyces,* including *Streptomyces griseovirides, Streptomyces lydicus,*
bacteria of the genus *Agrobacterium,* including *Agrobacterium radiobacter,*
bacteria of the genus *Erwinia,* including non-pathogenic *Erwinia carotovora,*
bacteria of the genus *Variovorax,* including *Variovorax paradoxus,*
bacteria of the genus *Lactobacillus,* including *Lactobacillus plantarum,*

c) other agents
possible elicitors, including yeast or its extract, β-glucan, chitin or chitosan or their fragments, β-aminobutyric acid, 2,6-dichloroisonicotinic acid, salicylic acid or its derivatives, algae extract, algae extract (hydrolysate), jasmine flower extract, sodium alginate, oligosaccharides, trehalose, polysaccharides, lipids, lipopolysaccharides, fatty acids, glycolipids, glycoproteins, glycopeptides, proteins or peptides derived from plants and/or pathogenic microorganisms, or ergosterol, and

(N) unclassified agents
mineral oils, organic oils, inorganic salts, material of natural origin, potassium bicarbonate, sodium hydrogen carbonate, calcium carbonate, calcium hydroxide, potassium iodide, potassium phosphonates, chitosan hydrochloride, and urea.

[3] The agricultural/horticultural fungicidal composition according to [1] or [2], wherein the component (II) is at least one component selected from the group consisting of azoxystrobin, ipflufenoquin, impyrfluxam, epoxiconazole, chlorothalonil, cyazofamid, difenoconazole, cyproconazole, tebuconazole, triflumizole, trifloxystrobin, bixafen, picoxystrobin, pyraclostrobin, fluazinam, fluxapyroxad, prothioconazole, florylpicoxamid, benzovindiflupyr, mancozeb, iminoctadine trialbesilate, cyflufenamid, cyprodinil, potassium bicarbonate, thiophanate-methyl, picarbutrazox, fludioxonil, metyltetraprole, mefentrifluconazole, copper sulfate, and metominostrobin.
[4] The agricultural/horticultural fungicidal composition according to any one of [1] to [3], wherein the component (I) and the component (II) have a weight ratio of 1:1,000,000 to 1,000,000:1.

## Effect of the Invention

An agricultural/horticultural fungicidal composition of the present invention has excellent disease control effects, causes no chemical damage to plant bodies, exerts less toxicity to humans, livestock, or fish, and less affects environment.

## Mode of Carrying Out the Invention

An agricultural/horticultural fungicidal composition of the present invention is a composition comprising component (I) and component (II).
The agricultural/horticultural fungicidal composition of the present invention, of course, may be applied as a mixture of the component (I) and component (II). An aspect, in which component (I) and component (II) are not mixed, but each component is applied separately, is included.
The component (I) in the present invention is at least one compound selected from the group consisting of a compound of formula (I) and a salt thereof. This compound may be used as an agricultural/horticultural fungicide.

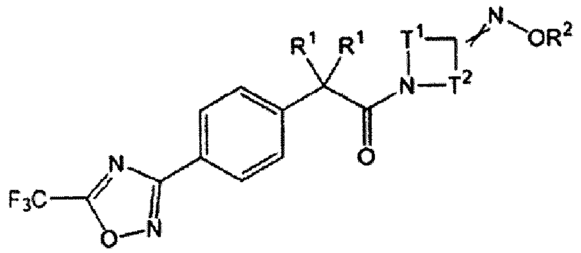

(I)

[wherein

$R^1$ each independently represents a hydrogen atom, a $C_{1-6}$ alkyl group, or a $C_{1-6}$ alkoxy group;
$R^2$ represents a $C_{1-6}$ alkyl group, a $C_{1-6}$ alkyl group having at least one $G^1$, a $C_{2-6}$ chained unsaturated hydrocarbon group, a $C_{2-6}$ chained unsaturated hydrocarbon group having at least one $G^1$, a $C_{3-6}$ cycloalkyl group, a $C_{3-6}$ cycloalkyl group having at least one $G^2$, a $C_{6-10}$ aryl group, a $C_{6-10}$ aryl group having at least one $G^2$, a 5- to 6-membered heterocyclyl group, or a 5- to 6-membered heterocyclyl group having at least one $G^2$;
$G^1$ each independently represents a halogeno group, a hydroxyl group, a $C_{1-6}$ alkoxy group, a $C_{1-6}$ haloalkoxy group, a $C_{1-6}$ alkylthio group, a $C_{1-6}$ alkylsulfinyl group, a $C_{1-6}$ alkylsulfonyl group, a $C_{3-6}$ cycloalkyl group, a $C_{6-10}$ aryl group, a $C_{6-10}$ aryl group having at least one $g^1$, a 5- to 6-membered heterocyclyl group, a 5- to 6-membered heterocyclyl group having at least one $g^1$, or a cyano group;
$G^2$ each independently represents a halogeno group, a $C_{1-6}$ alkyl group, a $C_{1-6}$ haloalkyl group, a hydroxyl group, a $C_{1-6}$ alkoxy group, a $C_{1-6}$ alkylthio group, a $C_{1-6}$ alkylsulfinyl group, a $C_{1-6}$ alkylsulfonyl group, a $C_{3-6}$ cycloalkyl group, a $C_{6-10}$ aryl group, a $C_{6-10}$ aryl group having at least one $g^1$, a 5- to 6-membered heterocyclyl group, a 5- to 6-membered heterocyclyl group having at least one $g^1$, a nitro group, or a cyano group;
$g^1$ each independently represents a halogeno group, a $C_{1-6}$ alkyl group, a $C_{1-6}$ haloalkyl group, a $C_{1-6}$ alkoxy group, a $C_{1-6}$ haloalkoxy group, a nitro group, or a cyano group;
$T^1$ represents a $C_{1-2}$ alkylene group or a $C_{1-6}$ alkylene group having at least one $X^4$;
$T^2$ represents a $C_{1-2}$ alkylene group or a $C_{1-6}$ alkylene group having at least one $X^4$; and
$X^4$ substituents each independently represent a halogeno group or a $C_{1-6}$ alkyl group.]

Here, each term used herein will be described.

The term "$C_{1-6}$ alkyl group" means a linear or branched $C_{1-6}$ saturated hydrocarbon group.

Specifically a methyl group, an ethyl group, an n-propyl group, an i-propyl group, an n-butyl group, an s-butyl group, an i-butyl group, a t-butyl group, an n-pentyl group, or an n-hexyl group or the like may be exemplified.

The term "$C_{2-6}$ chained unsaturated hydrocarbon group" means a linear or branched $C_{2-6}$ hydrocarbon group containing at least one double or triple bond. Examples include, in addition to a "$C_{2-6}$ alkenyl group" or a "$C_{2-6}$ alkynyl group", those having two or more double or triple bonds, such as a butadienyl group, a butadiynyl group, a pentadienyl group, or a pentadiynyl group.

The term "$C_{2-6}$ alkenyl group" means a linear or branched $C_{2-6}$ hydrocarbon group containing one double bond.

Specifically, a vinyl group, a 1-propenyl group, a 2-propenyl group (allyl group), a 1-butenyl group, a 2-butenyl group, a 3-butenyl group, a 1-methyl-2-propenyl group, or a 2-methyl-2-propenyl group or the like may be exemplified.

The term "$C_{2-6}$ alkynyl group" means a linear or branched $C_{2-6}$ hydrocarbon group containing one triple bond.

Specifically, an ethynyl group, a 1-propynyl group, a 2-propynyl group, a 1-butynyl group, a 2-butynyl group, a 3-butynyl group, or a 1-methyl-2-propynyl group or the like may be exemplified.

The term "$C_{3-6}$ cycloalkyl group" means a $C_{3-6}$ saturated monocyclic hydrocarbon group (also called monocyclic carbon ring group) with carbon atoms arranged in a ring.

Specifically, a cyclopropyl group, a cyclobutyl group, a cyclopentyl group, or a cyclohexyl group or the like may be exemplified.

The term "$C_{6-10}$ aryl group" means a $C_{6-10}$ monocyclic or bicyclic hydrocarbon group having carbon atoms arranged in a ring and at least one aromatic ring. The term is also called an aromatic group.

Specifically, a phenyl group or a naphthyl group or the like may be exemplified.

The term "5- to 6-membered heterocyclyl group" means a cyclic group consisting of 5 to 6 ring atoms and a saturated or partially unsaturated 5- to 6-membered heterocyclyl group in which 1 to 4 of the ring atoms have heteroatoms each independently selected from nitrogen, oxygen, or sulfur, or a cyclic group consisting of 5 to 6 ring atoms and a 5- to 6-membered heteroaryl group in which 1 to 4 of the ring atoms have heteroatoms each independently selected from nitrogen, oxygen, or sulfur.

Specifically, as the 5-membered saturated heterocyclyl group, a pyrrolidinyl group, a tetrahydrofuranyl group, or a thiazolidinyl group or the like may be exemplified.

As the 6-membered saturated heterocyclyl group, a piperidyl group, a piperazinyl group, a morpholinyl group, a tetrahydropyranyl group, a dioxolanyl group, or a dioxanyl group or the like may be exemplified.

As the 5-membered heteroaryl group, a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, or a tetrazolyl group or the like may be exemplified.

As the 6-membered heteroaryl group, a pyridyl group, a pyrazinyl group, a pyrimidinyl group, a pyridazinyl group, or a triazinyl group or the like may be exemplified.

As the 5-membered partially unsaturated heterocyclic group, a pyrrolinyl group, a dihydrofuranyl group, an imidazolinyl group, a pyrazolinyl group, an oxazolinyl group, or an isoxazolinyl group or the like may be exemplified.

As the 6-membered partially unsaturated heterocyclic group, a dihydropyranyl group or the like may be exemplified.

The term "halogeno group" means a fluoro group, a chloro group, a bromo group, or an iodo group.

The term "$C_{1-6}$ alkoxy group" means a group formed by bonding a $C_{1-6}$ alkyl group and a hydroxyl group.

Specifically, a methoxy group, an ethoxy group, an n-propoxy group, an i-propoxy group, an n-butoxy group, an s-butoxy group, an i-butoxy group, or a t-butoxy group or the like may be exemplified.

The term "$C_{1-6}$ alkylthio group" means a group formed by bonding a $C_{1-6}$ alkyl group and a thiol group.

Specifically, a methylthio group, an ethylthio group, an n-propylthio group, an i-propylthio group, an n-butylthio group, an i-butylthio group, an s-butylthio group, or a t-butylthio group or the like may be exemplified.

The term "$C_{1-6}$ alkylsulfinyl group" means a group formed by bonding a $C_{1-6}$ alkyl group and a sulfinyl group.

Specifically, a methyl sulfinyl group, an ethyl sulfinyl group, or a t-butyl sulfinyl group or the like may be exemplified.

The term "$C_{1-6}$ alkylsulfonyl group" means a group formed by bonding a $C_{1-6}$ alkyl group and a sulfonyl group.

Specifically, a methyl sulfonyl group, an ethyl sulfonyl group, or a t-butyl sulfonyl group or the like may be exemplified.

The term "$C_{1-6}$ haloalkyl group" means a halogeno-substituted $C_{1-6}$ alkyl group.

Specifically, a chloromethyl group, a chloroethyl group, a difluoromethyl group, a trifluoromethyl group, a 2,2,2-trifluoroethyl group, a 1-fluoro-n-butyl group, or a perfluoro-n-pentyl group or the like may be exemplified.

The term "$C_{1-6}$ haloalkoxy group" means a halogeno-substituted $C_{1-6}$ alkoxy group.

Specifically, a trifluoromethoxy group, a 2-chloro-n-propoxy group, a 2,3-dichlorobutoxy group, or a 2,2,2-trifluoroethoxy group or the like may be exemplified.

The term "$C_{1-2}$ alkylene group" means a divalent $C_{1-2}$ alkyl group.

Specifically, a methylene group, a dimethylene group or the like may be exemplified.

The term "$C_{1-6}$ alkylene group" means a divalent $C_{1-6}$ alkyl group.

Specifically, in addition to the above methylene or dimethylene group, a trimethylene group or a tetramethylene group or the like may be exemplified.

A salt of a compound of formula (I) is not particularly limited as long as the salt is agriculturally or horticulturally acceptable. For example, a salt of an inorganic acid (e.g., hydrochloric acid, sulfuric acid); a salt of an organic acid (e.g., acetic acid, lactic acid); a salt of an alkali metal (e.g., lithium, sodium, potassium); a salt of an alkali earth metal (e.g., calcium, magnesium); a salt of a transition metal (e.g., iron, copper); a salt of an organic base (e.g., triethylamine, tributylamine, pyridine, hydrazine); or ammonium.

Specific examples of the compound of formula (I) or the salt thereof include those listed in WO2019/022061, and they may be produced by known methods, such as the method described in the above publication.

The compounds listed in Table 1 below are particularly preferable examples.

[Table 1]

| Compound No. | |
|---|---|
| Compound 1 | 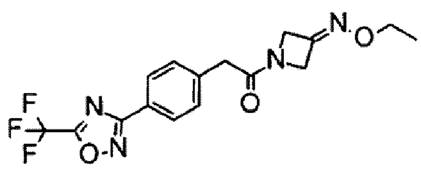 |

(continued)

| Compound No. | |
|---|---|
| Compound 2 | 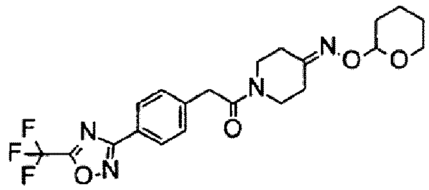 |
| Compound 3 | 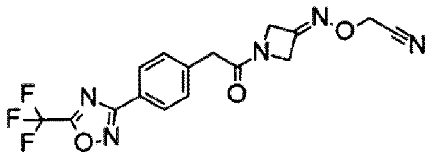 |
| Compound 4 | 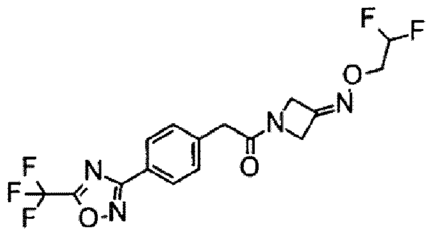 |
| Compound 5 | 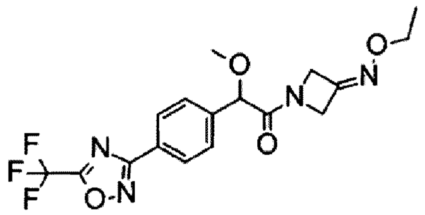 |
| Compound 6 | 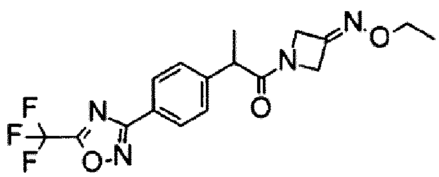 |
| Compound 7 | 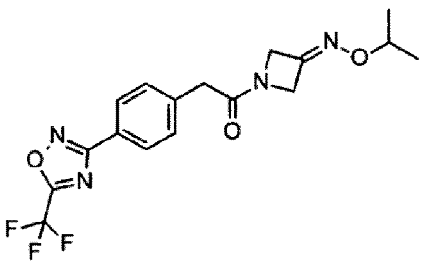 |
| Compound 8 | 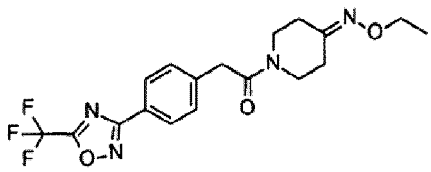 |

(continued)

| Compound No. | |
|---|---|
| Compound 9 | 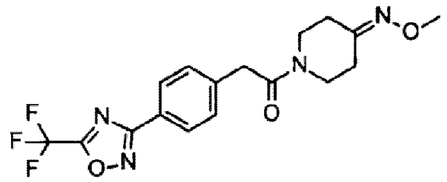 |
| Compound 10 | 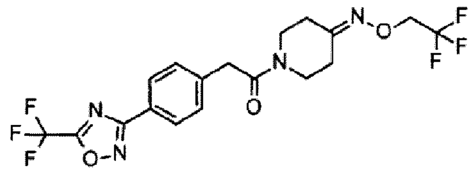 |
| Compound 11 | 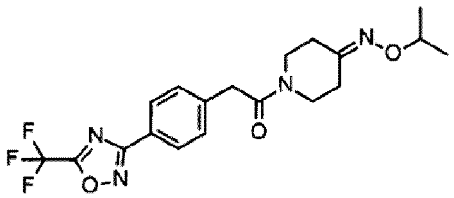 |
| Compound 12 | 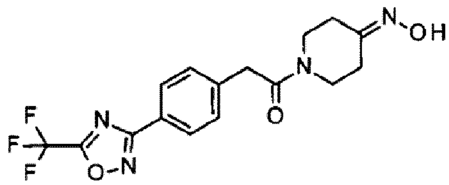 |

The component (II) used in combination with the component (I) in the present invention is, for instance, a compound (fungicide) that has fungicidal activity and is other than formula (I). The component (II) may also be any of a compound with insecticidal activity (insecticide), a compound with acaricidal activity (acaricide), a compound with herbicidal activity (herbicide), a compound with plant growth regulating activity (plant growth regulator), a fertilizer, or a compound with chemical damage reducing activity (chemical damage reducer). The component (II) may be at least one compound, and two or more compounds may be selected and used in combination.

The fungicide that may be used as the component (II) and the amount thereof are not particularly limited as long as its fungicidal effects can be exerted.

As the fungicide that may be used as the component (II), each compound listed on the FRAC (Fungicide Resistance Action Committee) website (https://www.frac.info/), or an agriculturally acceptable salt or derivative thereof may be exemplified.

Specific examples may include each compound selected from the following groups.

Fungicide:

(A) Agents acting on nucleic acid synthesis and metabolism:

A1) RNA polymerase I inhibitors
benalaxyl, benalaxyl-M, furalaxyl, metalaxyl, metalaxyl-M, oxadixyl, ofurace,
A2) adenosine deaminase inhibitors
bupirimate, dimethirimol, ethirimol,
A3) DNA/RNA biosynthesis inhibitors
hymexazole, octhilinone,
A4) DNA topoisomerase type II inhibitors

oxolinic acid.

(B) Agents acting on cytoskeleton and motor proteins:

B1) to B3) β-tubulin polymerization inhibitors benomyl, carbendazim, fuberidazole, thiabendazole, thiophanate, thiophanate-methyl, diethofencarb, zoxamide, ethaboxam, chlorfenazole, debacarb, trichlamide, zarilamid,
B4) mitosis (point of action unknown) inhibitors
pencycuron,
B5) delocalization inhibitors of spectrin-like proteins fluopicolide, fluopimomide,
B6) actin/myosin/fimbrin function inhibitors
phenamacril, metrafenone, pyriofenone.

(C) Agents acting on respiration:

C1) complex I:NADH oxidoreductase inhibitors
diflumetorim, tolfenpyrad, fenazaquin,
C2) complex II:succinate dehydrogenase inhibitors
benodanil, flutolanil, mepronil, isofetamid, fluopyram, cyclobutrifluram, fenfuram, carboxin, oxycarboxin, thifluzamide, benzovindiflupyr, bixafen, fluindapyr, fluxapyroxad, furametpyr, inpyrfluxam, isopyrazam, penflufen, penthiopyrad, sedaxane, isoflucypram, pydiflumetofen, boscalid, pyraziflumid, flubeneteram, furmecyclox,
C3) complex III:cytochrome bc1 (ubiquinol oxidase) Qo site (cyt b gene) inhibitors
azoxystrobin, coumoxystrobin, enoxastrobin, flufenoxystrobin, picoxystrobin, pyraoxystrobin, mandestrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, methyltetraprole,
C4) complex III:cytochrome bc1 (ubiquinone reductase) Qi site inhibitors
cyazofamid, amisulbrom, fenpicoxamid, florylpicoxamid, metarylpicoxamid,
C5) deconjugation inhibitors of oxidative phosphorylation binapacryl, dinocap, meptyldinocap, fluazinam,
C6) inhibitors of oxidative phosphorylation and ATP synthase fentin acetate, fentin chloride, fentin hydroxide,
C7) ATP transport inhibitors
silthiofam,
C8) complex III:cytochrome bc1 (ubiquinone reductase) Qo site, stigmatellin-binding subsite inhibitors
ametoctradin.

(D) Agents acting on amino acid and protein synthesis:

D1) methionine biosynthesis (cgs gene) inhibitors
cyprodinil, mepanipyrim, pyrimethanil,
D2) protein synthesis (ribosomal translation termination step) inhibitors
blasticidin-S,
D3), D4) protein synthesis (ribosomal translation initiation step) inhibitors
kasugamycin, kasugamycin hydrochloride, streptomycin,
D5) protein synthesis (ribosomal polypeptide elongation step) inhibitors
oxytetracycline.

(E) Agents acting on signal transduction:

E1) signal transduction (mechanism of action unknown) inhibitors
quinoxyfen, proquinazid,
E2) MAP/histidine kinase (os-2, HOG1) inhibitors in osmotic signal transduction
fenpiclonil, fludioxonil,
E3) MAP/histidine kinase (os-1, Daf1) inhibitors in osmotic signal transduction
clozolinate, dimethachlone, iprodione, procymidone, vinclozolin.

(F) Agents acting on lipid biosynthesis or transport/cell membrane structure or function:

F1) dicarboxyimide-based fungicides
F2) phospholipid biosynthesis, methyltransferase inhibitors edifenphos, iprobenfos, pyrazophos, isoprothiolane,
F3) cell peroxidation inhibitors

biphenyl, chloroneb, dicloran, quintozene, tecnazene, tolclofos-methyl, etridiazole,
F4) cell membrane permeability, fatty acid inhibitors
iodocarb, propamocarb, propamocarb hydrochloride, prothiocarb,
F5) carboxylic acid amide (CAA)-based fungicides
F8) ergosterol binding inhibitors
natamycin,
F9) lipid homeostasis and transport/storage inhibitors oxathiapiprolin, fluoxapiprolin.

(G) Inhibitors of sterol biosynthesis in cell membrane

G1) demethylase (erg11/cyp51) inhibitors at C14 position of sterol biosynthesis
triforine, pyrifenox, pyrisoxazole, fenarimol, nuarimol, imazalil, oxpoconazole, pefurazoate, prochloraz, triflumizole, azaconazole, bitertanol, bromuconazole, cyproconazole, difenoconazole, diniconazole, epoxiconazole, etaconazole, fenbuconazole (enbuconazole), fluquinconazole, flusilazole, flutriafol, hexaconazole, imibenconazole, ipconazole, mefentrifluconazole, metconazole, myclobutanil, penconazole, propiconazole, simeconazole, tebuconazole, tetraconazole, triadimefon, triadimenol, triticonazole, prothioconazole, fluoxytioconazole, furconazole, furconazole-cis, diniconazole-M,
G2) Δ14-reductase and Δ8→Δ7-isomerase (erg24, erg2) inhibitors in sterol biosynthesis
aldimorph, dodemorph, dodemorph acetate, fenpropimorph, tridemorph, fenpropidin, piperalin, spiroxamine, buthiobate,
G3) 3-keto reductase (erg27) inhibitors in C4-position demethylation of sterol biosynthetic system
fenhexamid, fenpyrazamine,
G4) squalene epoxidase (erg1) inhibitors of sterol biosynthesis system
pyributicarb, naftifine, terbinafine.

(H) Cell wall biosynthesis inhibitors:

H4) chitin synthase inhibitors
polyoxin, polyoxorim,
H5) cellulose synthase inhibitors
dimethomorph, flumorph, pyrimorph, benthiavalicarb, iprovalicarb, valifenalate, mandipropamid.

(I) Cell wall melanin synthesis inhibitors:

I1) reductase inhibitors in melanin biosynthesis
fthalide, pyroquilon, tricyclazole,
I2) dehydratase inhibitors in melanin biosynthesis
carpropamid, diclocymet, fenoxanil,
I3) polyketide synthase inhibitors in melanin biosynthesis tolprocarb.

(P) Agents acting to induce resistance in host plants:

P01 to P03) agents involving salicylic acid signal transduction
acibenzolar-S-methyl, probenazole, tiadinil, isotianil,
P04) polysaccharide elicitors
laminarin,
P05) anthraquinone elicitors
extract from *Reynoutria sachalinensis,*
P06) microbial elicitors
cell walls of *Bacillus mycoides* isolate J, *Saccharomyces cerevisiae* strain LAS117,
P07) phosphonates
fosetyl-Al or phosphorous acids and salts such as phosphorous acid or calcium phosphite, aluminum phosphite, sodium phosphite, disodium phosphonate,
P08) agents involving salicylic acid signal transduction dichlobentiazox.

(U) Agents with unknown mechanism of action:
cymoxanil, teclofthalam, triazoxide, flusulfamide, diclomezine, cyflufenamid, dodine, dodine free base, flutianil, ferimzone, tebufloquin, picarbutrazox, validamycin, bethoxazin, cyprofuram, flumetover, nitrothal-isopropyl, propamidine, ipflufenoquin, pyridachlometyl, pyrapropoyne, aminopyrifen, ipfentrifluconazole, quinofumelin, dipymetitrone, chloroinconazide, seboctylamine, flumetylsulforim, flufenoxadiazam.

(M) Agents with multiple action point contact activity:

copper (different salts), basic copper sulfate, Bordeaux mixture, copper hydroxide, copper naphthenate, copper oxychloride, copper sulfate, cuprous oxide, oxine-copper, sulfur, lime sulfur, amobam, ferbam, mancozeb, maneb, metiram, propineb, thiram, zinc thiazole, zineb, ziram, captan, captafol, folpet, chlorothalonil, dichlofluanid, tolylfluanid; guazatine, guazatine acetates, iminoctadine, iminoctadine triacetate, iminoctadine trialbesilate, anilazine, dithianon, chinomethionat/quinomethionate, fluoroimide, methasulfocarb, dazomet, cufraneb, mancopper, polycarbamate.

(BM) Biological pesticides/biological -derived pesticides with multiple mechanisms of action

a) plant extracts:

polypeptides (lectin), phenols, sesquiterpenes, triterpenoids, coumarins, terpene hydrocarbons, terpene alcohols, terpene phenols, extract from the cotyledons of lupine plantlets, extract from *Swinglea glutinosa,* extract from *Melaleuca alternifolia* (tea tree oil), plant oils (mixtures), eugenol, geraniol, thymol, alpha-pinene, alpha-terpinene, alpha-terpinol, alpha-terpinoline, gamma-terpinene, d-limonene, orange oil, linalool, menthol, ursolic acid, oleanolic acid, neem oil,

b) microorganisms (microbial strains or their extracts or metabolites):

fungi of the genus *Trichoderma (Trichoderma* spp.) such as *Trichoderma atroviride* strain I-1237, *Trichoderma atroviride* strain LU132, *Trichoderma atroviride* strain SC1, *Trichoderma atroviride* strain SKT-1, *Trichoderma atroviride* strain 77B; *Trichoderma asperellum* strain T34, *Trichoderma asperellum* strain kd; *Trichoderma harzianum* strain T-22; *Trichoderma virens* strain G-41,

fungi of the genus *Gliocladium* (*Gliocladium* spp.) such as *Gliocladium catenulatum* strain J1446,

fungi of the genus *Clonostachys* (*Clonostachys* spp.) such as *Clonostachys rosea* strain CR-7,

fungi of the genus *Coniothyrium* (*Coniothyrium* spp.) such as *Coniothyrium minitans* strain CON/M/91-08,

fungi of the genus *Talaromyces* (*Talaromyces* spp.) such as *Talaromyces flavus* strain SAY-Y-94-01,

yeast of the genus *Saccharomyces* (*Saccharomyces* spp.) such as *Saccharomyces cerevisiae* strain LAS02,

bacteria of the genus *Bacillus* (*Bacillus* spp.) such as *Bacillus amyloliquefaciens* strain QST713, *Bacillus amyloliquefaciens* strain FZB24, *Bacillus amyloliquefaciens* strain MBI600, *Bacillus amyloliquefaciens* strain D747, *Bacillus amyloliquefaciens* strain F727, *Bacillus amyloliquefaciens* strain AT-332, *Bacillus subtilis* strain AFS032321, *Bacillus subtilis* strain Y1336, *Bacillus subtilis* strain HAI-0404, *Bacillus simplex,*

bacteria of the genus *Paenibacillus* (*Paenibacillus* spp.), bacteria of the genus *Burkholderia* (*Burkholderia* spp.), fungi of the genus *Fusarium* (*Fusarium* spp.),

bacteria of the genus *Pseudomonas* (*Pseudomonas* spp.) such as *Pseudomonas chlororaphis* strain AFS009, *Pseudomonas fluorescens, Pseudomonas rhodesiae* HAI-0804,

bacteria of the genus *Streptomyces* (*Streptomyces* spp.) such as *Streptomyces griseovirides* strain K61, *Streptomyces lydicus* strain WYEC108,

bacteria of the genus *Agrobacterium* (*Agrobacterium* spp.) such as *Agrobacterium radiobacter,*

bacteria of the genus *Erwinia* (*Erwinia* spp.) such as non-pathogenic *Erwinia carotovora* subsp. *carotovora,*

bacteria of the genus *Varioborax* (*Variovorax* spp.) such as *Variovorax paradoxus,*

bacteria of the genus *Lactobacillus* (*Lactobacillus* spp.) such as *Lactobacillus plantarum,*

c) other agents

possible elicitors, such as yeast or its extract, β-glucan, chitin or chitosan or their fragments, β-aminobutyric acid, 2,6-dichloroisonicotinic acid, salicylic acid or its derivatives, algae extract, algae extract (hydrolysate), jasmine flower extract, sodium alginate, oligosaccharides, trehalose, polysaccharides, lipids, lipopolysaccharides, fatty acids, glycolipids, glycoproteins, glycopeptides, proteins or peptides derived from plants and/or pathogenic microorganisms, or ergosterol.

(N) Unclassified agents

mineral oils, organic oils, inorganic salts, material of biological origin, potassium bicarbonate, sodium hydrogen carbonate, calcium carbonate, calcium hydroxide, potassium iodide, potassium phosphonates, chitosan hydrochloride, urea.

The component (II) may be an insecticide or acaricide. As preferable the insecticide or acaricide that may be used as the component (II),

each compound listed on the IRAC (Insecticide Resistance Action Committee) website (https://irac-online.org/), or an

agriculturally acceptable salt or derivative thereof may be exemplified.

Specific examples may include each compound selected from the following groups.

Insecticides and acaricides:

(1A) Acetylcholinesterase (AChE) inhibitors (carbamate-based ones):
alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC, xylylcarb, aldoxycarb, allyxycarb, aminocarb, bufencarb, cloethocarb, fenothiocarb, promecarb.
(1B) Acetylcholinesterase (AChE) inhibitors (organophosphorous-based ones):
acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos (DDVP), dicrotophos, dimethoate, dimethylvinphos,disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl)salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-e, cyanofenphos, demeton-S-methylsulfone, dialifos, dichlofenthion, dioxabenzofos, etrimfos, fensulfothion, fonofos, formothion, iodofenphos, isazofos, isocarbofos, methacrifos, phosphocarb, pirimiphos-ethyl (pirimiphos-e), propaphos, prothoate, sulprofos.
(2) GABAergic chloride ion (chlorine ion) channel blockers: chlordane, endosulfan, ethiprole, fipronil, acetoprole, camphechlor, dienochlor, heptachlor, pyrafluprole, pyriprole, flufiprole.
(3A) Sodium channel modulators (pyrethroid-based ones): acrinathrin, allethrin, d-cis-trans-allethrin, d-trans-allethrin, bifenthrin, bioallethrin, bioallethrin-S-cyclopentenyl-isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin[(1R)-trans-isomers], deltamethrin, empenthrin[(EZ)-(1R)-isomers], esfenvalerate, etofenprox, fenpropathrin, fenvalerate, flucythrinate, flumethrin, tau-fluvalinate, halfenprox, imiprothrin, kadethrin, permethrin, phenothrin[(1R)-trans-isomer], prallethrin, pyrethrins, resmethrin, silafluofen, tefluthrin, tetramethrin, tetramethrin[(1R)-isomers], tralomethrin, transfluthrin, kappa-bifenthrin, biopermethrin, chloroprallethrin, dimefluthrin, fenfluthrin, fenpirithrin, flufenprox, heptafluthrin, meperfluthrin, epsilon-metofluthrin, momfluorothrin, epsilon-momfluorothrin, trans-permethrin, profluthrin, protrifenbute, kappa-tefluthrin, terallethrin, tetramethylfluthrin, bioethanomethrin.
(3B) Sodium channel modulators (DDT compounds):
DDT, methoxychlor.
(4) Nicotinergic acetylcholine receptor (nAChR) competitive modulators:
acetamiprid, clothianidin, dinotefuran, imidacloprid, nitenpyram, thiacloprid, thiamethoxam, nicotine, sulfoxaflor, flupyradifurone, triflumezopyrim, nithiazine, dicloromezotiaz, flupyrimin.
(5) Nicotinergic acetylcholine receptor (nAChR) allosteric modulators:
spinetoram, spinosad.
(6) Glutamatergic chloride ion (chlorine ion) channel (GluCl) allosteric modulators:
abamectin, emamectin, emamectin-benzoate, lepimectin, milbemectin, doramectin, eprinomectin, ivermectin, moxidectin, selamectin.
(7) Juvenile hormone analogues:
hydroprene, kinoprene, methoprene, fenoxycarb, pyriproxifen, diofenolan, epofenonane, triprene.
(8) Other nonspecific (multi-site) inhibitors:
alkyl halides (e.g., methyl bromide), chloropicrin, sodium aluminum fluoride, sulfuryl fluoride, borax, boric acid, disodium octaborate, sodium borate, sodium metaborate, tartar emetic, dazomet, metam, metam potassium, metam sodium.
(9) String organ TRPV channel modulators:
pymetrozine, pyrifluquinazon, afidopyropen.
(10) Mite growth inhibitors:
clofentezine, diflovidazin, hexythiazox, etoxazole.
(11) Microbially derived insect enteric membrane disruptors:
*B.t.* subsp. *israelensis, B.t.* subsp. *aizawai, B.t.* subsp. *kurstaki, B.t.* subsp. *tenebrionis, B.t.* crop proteins (e.g.,

Cry1Ab, Cry1Ac, Cry1Fa, Cry1A.105, Cry2Ab, Vip3A, mCry3A, Cry3Ab, Cry3Bb, Cry34Ab1/Cry35Ab1), *Bacillus sphaericus*. Note: "B.t." refers to *Bacillus thuringiensis,* and "B.t. crop proteins" refers to proteins produced in crops in which genes for toxin proteins (e.g., Cry1Ab) produced by *B.t.* (*Bacillus thuringiensis*) have been integrated.
(12) Mitochondrial ATP synthase inhibitors:
diafenthiuron, azocyclotin, cyhexatin, fenbutatin-oxide, propargite, tetradifon.
(13) Oxidative phosphorylation deconjugation agents that disturb the proton gradient:
chlorfenapyr, DNOC (4,6-dinitro-o-cresol), sulfluramid, binapacryl, dinobuton, dinocap.
(14) Nicotinergic acetylcholine receptor (nAChR) channel blockers:
bensultap, cartap hydrochloride, thiocyclam, thiosultap-sodium.
(15) Chitin biosynthesis inhibitors, type 0:
bistrifluron, chlorfluazuron, diflubenzuron, flucycloxuron, flufenoxuron, hexaflumuron, lufenuron, novaluron, noviflumuron, teflubenzuron, triflumuron, fluazuron.
(16) Chitin biosynthesis inhibitors, type 1:
buprofezin.
(17) Ecdysis inhibitors
cyromazine.
(18) Ecdysis hormone (ecdysone) receptor agonists:
chromafenozide, halofenozid, methoxyfenozide, tebufenozide.
(19) Octopamine receptor agonists:
amitraz, chlordimeform.
(20) Mitochondrial electron transfer complex III Inhibitors:
hydramethylnon, acequinocyl, fluacrypyrim, bifenazate.
(21) Mitochondrial electron transfer complex I Inhibitors (METI):
fenazaquin, fenpyroximate, pyridaben, pyrimidifen, tebufenpyrad, tolfenpyrad, rotenone.
(22) Potential-dependent sodium channel blockers:
indoxacarb, metaflumizone.
(23) Acetyl CoA carboxylase inhibitors:
spirodiclofen, spiromesifen, spirotetramat, spiropidion.
(24) Mitochondrial electron transfer complex IV Inhibitors: Al-phosphide, Ca-phosphide, Zn-phosphide, phosphine, calcium cyanide (Ca-cyanide), sodium cyanide (Na-cyanide), potassium cyanide (K-cyanide).
(25) Mitochondrial electron transfer complex II Inhibitors: cyenopyrafen, cyflumetofen, pyflubumide.
(26) Ryanodine receptor modulators:
chlorantraniliprole, cyantraniliprole, cyclaniliprole, flubendiamide, cyhalodiamide, tetrachlorantraniliprole, tetraniliprole.
(27) String organ modulators (target site unspecified):
flonicamid.
(28) GABAergic chloride ion (chlorine ion) channel allosteric modulators:
broflanilide, fluxametamide, isocycloseram, afoxolaner, fluralaner, lotilaner, sarolaner.
(29) Agents with unknown mechanism of action (UN):
azadirachtin, benzoximate, bromopropylate, chinomethionat/quinomethionate, dicofol, lime sulfur, mancozeb, pyridalyl, sulfur.
(30) Other insecticides and acaricides:
acynonapyr, amidoflumet, benzomate, benzpyrimoxan, chlorobenzilate, dicyclanil, fenoxacrim, fentrifanil, flometoquin, flubenzimine, flufenzine, fluhexafon, fluopyram, metaflumizone, metoxadiazone, oxazosulfyl, tetrasul, triarathene, tyclopyrazoflor.

The component (II) may be a herbicide. As the herbicide that may be used as the component (II), each compound listed on the HRAC (Herbicide Resistance Action Committee) website (https://hracglobal.com/), or an agriculturally acceptable salt or derivative thereof may be exemplified.
Specific examples may include each compound selected from the following groups.

Herbicides:

(1) Herbicides belonging to the group of acetyl CoA carboxylase (ACCase) inhibitors:
clodinafop-propargyl, clofop, cyhalofop-butyl, diclofop-methyl, fenoxaprop-ethyl, fenthiaprop, fluazifop-butyl, haloxyfop-methyl, isoxapyrifop, metamifop, quizalofop-ethyl, alloxydim, butroxydim, clethodim, cloproxydim, cycloxydim,

profoxydim, sethoxydim, tepraloxydim, tralkoxydim, pinoxaden, propaquizafop, fluazifop.

(2) Herbicides belonging to the group of acetolactate synthase (ALS) inhibitors (acetohydroxy acid synthase (AHAS) inhibitors):

imazamethabenz-methyl, imazamox, imazapic, imazapyr, imazaquin, imazethapyr, bispyribac-sodium, pyribenzoxim (prodrug of bispyribac), pyriftalid, pyriminobac-methyl, pyrithiobac-sodium, pyrimisulfan, triafamone, amidosulfuron, azimsulfuron, bensulfuron-methyl, chlorimuron-ethyl, chlorsulfuron, cinosulfuron, cyclosulfamuron, ethametsulfuron-methyl, ethoxysulfuron, flazasulfuron, flucetosulfuron, flupyrsulfuron-methyl-Na, foramsulfuron, halosulfuron-methyl, imazosulfuron, iodosulfuron-methyl-Na, mesosulfuron-methyl, metazosulfuron, metsulfuron-methyl, nicosulfuron, orthosulfamuron, oxasulfuron, primisulfuron-methyl, propyrisulfuron, prosulfuron, pyrazosulfuron-ethyl, rimsulfuron, sulfometuron-methyl, sulfosulfuron, thifensulfuron-methyl, triasulfuron, tribenuron-methyl, trifloxysulfuron-Na, triflusulfuron-methyl, tritosulfuron, flucarbazone, flucarbazone-Na, propoxycarbazone-Na, thiencarbazone-methyl, cloransulam-methyl, diclosulam, florasulam, flumetsulam, metosulam, penoxsulam, pyroxsulam, iodosulfuron-methyl, monosulfuron-methyl, iofensulfuron, metosulfam, mesosulfuron, trifloxysulfuron.

(3) Herbicides belonging to the group of microtubule polymerization inhibitors:

propyzamide=pronamide, tetrachlorothiophene (TCTP), chlorthal-dimethyl (DCPA), benefin=benfluralin, butralin, dinitramine, ethalfluralin, fluchloralin, isopropalin, nitralin, oryzalin, pendimethalin, prodiamine, profluralin, trifluralin, butamifos, DMPA, dithiopyr, thiazopyr, chlorthal.

(4) Herbicides belonging to the group of indoleacetic acid-like activators (synthetic auxin):

chloramben, MDBA (dicamba), TCBA (2,3,8-TBA) (TBA), benazolin-ethyl, 2,4,5-T, 2,4-PA (2,4-D), 2,4-DB, 2,4-D 2-ethylhexyl ester, 2,4-D amine, clomeprop, dichlorprop, fenoprop, MCPA, MCPB, MCPP (Mecoprop), chlorfenac=fenac, chlorfenprop, aminopyralid, clopyralid, florpyrauxifen, halauxifen, halauxifen-methyl, picloram, fluroxypyr, triclopyr, aminocyclopyrachlor, quinclorac, quinmerac, chlorflurenol, chlorflurenol-methyl, benazolin.

(5) Herbicides belonging to the group of photosynthesis (photosystem II) inhibitors - serine 264 binders:

chloranocryl=dicryl, CMMP (pentanochlor), DCPA (propanil), chlorprocarb, desmedipham, phenisopham, phenmedipham, brompyrazon, PAC (=chloridazon (=pyrazon)), ametryne, atraton, atrazine, aziprotryne=aziprotryn, chlorazine, CP 17029, cyanazine, cyprazine, desmetryne, dimethametryn, dipropetryn, eglinazine-ethyl, ipazine, methoprotryne=methoprotryn, procyazine, proglinazine-ethyl, prometon, prometryne, propazine, sebuthylazine, secbumeton, CAT (simazine), simetryne, terbumeton, terbuthylazine, terbutryne, trietazine, ethiozin, hexazinone, isomethiozin, metamitron, metribuzin, amicarbazone, bromacil, isocil, lenacil, terbacil, benzthiazuron, bromuron, buturon, chlorbromuron, chlorotoluron, chloroxuron, difenoxuron, dimefuron, DCMU (diuron), ethidimuron, fenuron, fluometuron, fluothiuron, isoproturon, isouron, linuron, methabenzthiazuron, metobenzuron, metobromuron, metoxuron, monolinuron, CMU (monuron), neburon, parafluron, siduron, tebuthiuron, thiazafluron, cypromid, cybutryne, karbutilate.

(6) Herbicides belonging to the group of photosynthesis (photosystem II) inhibitors - histidine 215 binders:

bentazon, bromofenoxim, bromoxynil, ioxynil, pyridate, pyridafol.

(7) Herbicides belonging to the group of 5-enolpyruvylshikimic acid-3-phosphate (EPSP) synthase inhibitors:

glyphosate.

(8) Herbicides belonging to the group of glutamine synthase inhibitors:

bialaphos/bilanafos, glufosinate-ammonium, glufosinate.

(9) Herbicides belonging to the group of chlorosis:carotenoid biosynthetic pathway phytoene desaturase (PDS) inhibitors:

fluridone, flurtamone, flurochloridone, norflurazon, beflubutamid, diflufenican, picolinafen, metflurazon.

(10) Herbicide belonging to the group of chlorosis: 1-deoxy-D-xylulose-5-phosphate (DOXP) synthase inhibitors:

bixlozone, clomazone.

(11) Herbicides belonging to the group of protoporphyrinogen oxidase (PPO) inhibitors:

acifluorfen, bifenox, chlomethoxyfen, CNP (chlornitrofen), fluorodifen, fluoroglycofen-ethyl, CFNP (fluoronitrofen), fomesafen, lactofen, NIP (nitrofen), oxyfluorfen, butafenacil, chlorphthalim, cinidon-ethyl, flumiclorac-pentyl, flumioxazin, flumipropyn, fluthiacet-methyl, pentoxazone, saflufenacil, tiafenacil, trifludimoxazin, oxadiargyl, oxadiazon, azafenidin, carfentrazone-ethyl, sulfentrazone, pyraclonil, pyraflufen-ethyl, epyrifenacil, halosafen, ethoxyfen-ethyl, thidiazimin, benzfendizone, profluazol, flufenpyr-ethyl, bencarbazone.

(12) Herbicides belonging to the group of very long chain fatty acid synthesis (VLCFAs) inhibitors:

cafenstrol, fentrazamide, ipfencarbazone, benfuresate, ethofumesate, fenoxasulfone, pyroxasulfone, indanofan, tridiphane, butylate, hexylthiocarbam (cycloate), dimepiperate, EPTC, esprocarb, molinate, orbencarb, pebulate, prosulfocarb, thiobencarb (=benthiocarb), tiocarbazil, tri-allate, vernolate, acetochlor, alachlor, CDAA (Allidochlor=CDAA), butachlor, butenachlor, delachlor, diethatyl-ethyl, dimethachlor, dimethenamid, dimethenamid-P, metazachlor, metolachlor, S-metolachlor, pethoxamid, pretilachlor, propachlor, propisochlor, prynachlor, thenylchlor, flufenacet, mefenacet, anilofos, piperophos, diallate, dimesulfazet.

(13) Herbicides belonging to the group of DHP (dihydropteroic acid) synthase inhibitors:

asulam.
(14) Herbicides belonging to the group of auxin transfer inhibitors:
diflufenzopyr-sodium, NPA (naptalam), diflufenzopyr.
(15) Herbicides belonging to the group of photosystem I electron conversion agents:
cyperquat, diquat, morfamquat, paraquat.
(16) Herbicides belonging to the group of mitotic/microtubule formation inhibitors:
barban, carbetamide, chlorbufam, IPC (chlorpropham), propham, swep.
(17) Herbicides belonging to the group of uncoupling (membrane-disrupting) agents:
dinosam, DNBP (dinoseb), dinoterb, DNOC, etinofen, medinoterb.
(18) Herbicides belonging to the group of chlorosis:4-hydroxyphenylpyruvate dioxygenase (4-HPPD) inhibitors:
isoxaflutole, benzofenap, pyrasulfotole, pyrazolynate, pyrazoxyfen, tolpyralate, topramezone, bicyclopyrone, fenquinotrione, mesotrione, sulcotrione, tefuryltrione, tembotrione, benzobicyclon, isoxachlortole, methoxyphenone, ketospiradox, tripyrasulfone, fenpyrazone, dioxopyritrione, cypyrafluone, bipyrazone, benquitrione, lancotrione sodium salt.
(19) Herbicides belonging to the group of cell wall (cellulose) synthesis inhibitors:
indaziflam, triaziflam, isoxaben, DCBN (chlorthiamid), DBN (dichlobenil), flupoxam.
(20) Herbicides belonging to the group of fatty acid thioesterase inhibitors:
cinmethylin, methiozolin.
(21) Herbicides belonging to the group of serine-threonine protein phosphatase inhibitors:
endothal.
(22) Herbicides belonging to the group of solanesyl diphosphate synthase inhibitors:
aclonifen.
(23) Herbicides belonging to the group of homogentisic acid solanesyltransferase inhibitors:
cyclopyrimorate.
(24) Herbicides belonging to the group of lycopene cyclase inhibitors:
ATA (amitrole).
(25) Herbicides belonging to the group of dihydroorotate dehydrogenase (DHODH) inhibitors: tetflupyrolimet.
(26) Other herbicides with unknown mechanism of action:
diphenamid, naproanilide, napropamide, flamprop-m, tebutam, SAP (bensulide), DPA (dalapon), tetrapion (flupropanate), TCA, mefluidide, perfluidone, bromobutide, cumyluron, difenzoquat, DSMA, dymron=daimuron, etobenzanid, fosamine, methyldymron, monalide, MSMA, oleic acid, oxaziclomefone, pelargonic acid, pyributicarb, ACN (quinoclamine), tetflupyrolimet, flamprop-isopropyl, dazomet, sodiumchlorate, CAMA, cacodylic acid, metam, rimisoxafen, cyclopyranil, clacyfos.

The component (II) may also be a plant growth regulator.

Specifically, as the plant growth regulator that may be used as the component (II), each compound selected from the following group may be exemplified.
abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetyl aminoethoxyvinyl glycine (a.k.a. aviglycine), aminooxyacetate, silver nitrate, cobalt chloride, IAA, 4-CPA, cloprop, 2,4-D, MCPB, indole-3-butyrate, dichlorprop, phenothiol, 1-naphthyl acetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyladenine, 5-aminolevulinic acid, daminozide.

The component (II) may be a fertilizer.

Specifically, as the fertilizer that may be used as the component (II), each compound selected from the following group may be exemplified.
ammonium sulfate ($(NH_4)_2SO_4$), magnesium sulfate ($MgSO_4$), ammonium phosphate ($(NH_4)_2HPO_4$), ammonium nitrate ($NH_4NO_3$), urea ($(NH_2)_2CO$), nitrogen (N), phosphorus (P), potassium (K).

The component (II) may be a chemical damage reducer (safener).

Specifically, as the chemical damage reducer that may be used as the component (II), each compound selected from the following group may be exemplified.
benoxacor, cloquintocet, cloquintocet-mexyl, cyometrinil, cyprosulfamide, dichlormid, dicyclonon, dietholate, fenchlorazole, fenchlorazole-ethyl, fenclorim, flurazole, fluxofenim, furilazole, isoxadifen, isoxadifen-ethyl, mefenpyr, mefenpyr-diethyl, mephenate, naphthalic anhydride, oxabetrinil, etc.

In the agricultural/horticultural fungicidal composition of the present invention, the ratio between component (I) and component (II) may be optionally selected. The weight ratio between component (I) and component (II) is usually from 1:1,000,000 to 1,000,000:1, preferably from 1:100,000 to 100,000:1, more preferably from 1:10,000 to 10,000:1,

still more preferably from 1:1,000 to 1,000:1, and still more preferably from 300:1 to 1:300, and particularly preferably from 1:100 to 100:1.

The agricultural/horticultural fungicidal composition of the present invention may be used for the control of plant diseases caused by a wide range of filamentous fungi, such as *Oomycetes, Ascomycetes, Deuteromycetes, Basidiomycetes,* or *Zygomycetes.*

Examples of the plant disease (pathogen), which is subject to control, are listed below.

Sugar beet: brown spot (*Cercospora beticola),* Aphanomyces root rot (*Aphanomyces cochlioides*), root rot (*Thanatephorus cucumeris*), leaf blight (*Thanatephorus cucumeris*), rust (*Uromyces betae*), powdery mildew (*Oidium* sp.), bacterial blight (*Ramularia beticola*), damping off (*Aphanomyces cochlioides, Pythium ultimum*), etc.

Peanut: brown leaf spot (*Mycosphaerella arachidis*), blotch (*Ascochyta* sp.), rust (*Puccinia arachidis),* standing blight (*Pythium debaryanum*), rust spot (*Alternaria alternata*)*,* southern blight (*Sclerotium rolfsii*), leaf spot (*Mycosphaerella berkeleyi*), peg, pod, and root necrosis (*Calonectria ilicicola*)*,* etc.

Cucumber: powdery mildew (*Sphaerotheca fuliginea),* downy mildew (*Pseudoperonospora cubensis*)*,* vine blight (*Mycosphaerella melonis*), vine split blight (*Fusarium oxysporum*), stem rot *(Sclerotinia sclerotiorum),* gray mold (*Botrytis cinerea*), anthracnose (*Colletotrichum orbiculare*), scab (*Cladosporium cucumerinum*), Corynespora leaf spot (*Corynespora cassiicola*), seedling blight (*Pythium debaryanum, Rhizoctonia solani Kuhn*), Phomopsis root rot *(Phomopsis* sp.), bacterial spot (*Pseudomonas syringae* pv. *Lachrymans*)*,* etc.

Tomato: gray mold (*Botrytis cinerea),* leaf mold (*Cladosporium fulvum*)*,* late blight (*Phytophthora infestans*)*,* Verticillium wilt (*Verticillium albo-atrum, Verticillium dahliae*), powdery mildew (*Oidium neolycopersici),* early blight (*Alternaria solani*)*,* Cercospora leaf mold (*Pseudocercospora fuligena*), bacterial wilt (*Ralstonia solanacearum*), stem rot *(Sclerotinia sclerotiorum),* etc.

Eggplant: gray mold *(Botrytis cinerea),* black blight (*Corynespora melongenae*)*,* powdery mildew (*Erysiphe cichoracearum*), leaf mold (*Mycovellosiella nattrassii*)*,* stem rot *(Sclerotinia sclerotiorum),* Verticillium wilt (*Verticillium dahliae*)*,* Phomopsis blight (*Phomopsis vexans),* etc.

Capsicum annuum: late blight (*Phytophthora capsici*)*,* gray mold *(Botrytis cinerea),* stem rot *(Sclerotinia sclerotiorum*)*,* anthracnose (*Colletotrichum aenigma, Colletotrichum capsici, Colletotrichum fructicola, Colletotrichum jiangxiense*), powdery mildew (*Leveillula taurica*), etc.

Strawberry: gray mold (*Botrytis cinerea*), powdery mildew (*Sphaerotheca humuli),* anthracnose *(Colletotrichum acutatum, Colletotrichum fragariae),* Phytophthora rot (*Phytophthora cactorum*), soft rot (*Rhizopus stolonifer*), Fusarium wilt (*Fusarium oxysporum*), Verticillium wilt (*Verticillium dahliae*), stem rot *(Sclerotinia sclerotiorum),* etc.

Onion: gray-mold neck rot (*Botrytis allii*), gray mold (*Botrytis cinerea),* leaf blight (*Botrytis squamosa),* downy mildew (*Peronospora destructor)*, Phytophthora rot (*Phytophthora porri*)*,* leaf blight (*Ciborinia allii*)*,* small sclerotial, neck rot (*Botrytis squamosa*)*,* Fusarium basal rot (*Fusarium oxysporum*)*,* pink root rot (*Pyrenochaeta terrestris*), white rot (*Sclerotium cepivorum*)*,* rust (*Puccinia allii*)*,* southern blight (*Sclerotium rolfsii),* etc.

Green onion: bacterial soft rot (*Pectobacterium carotovorum*)*,* downy mildew (*Peronospora destructor),* leaf blight (*Pleospora allii*), white rot (*Sclerotium cepivorum*)*,* rust (*Puccinia allii*), leaf blight (*Botrytis squamosa*), southern blight (*Sclerotium rolfsii*), pink root rot (*Pyrenochaeta terrestris*), etc.

Cabbage: clubroot (*Plasmodiophora brassicae*), bacterial soft rot (*Erwinia carotovora*), black rot (*Xanthomonas campesrtis* pv. *campestris*), bacterial leaf spot (*Pseudomonas syringae* pv. *maculicola, P.s.* pv. *alisalensis*), downy mildew (*Peronospora parasitica*), stem rot *(Sclerotinia sclerotiorum),* Alternaria sooty spot (*Alternaria brassicicola*), gray mold *(Botrytis cinerea),* black leg (*Phoma lingam*), Pythium rot *(Pythium aphanidermatum, Pythium ultimum),* white rust (*Albugo macrospora),* etc.

Lettuce: bacterial rot (*Pseudomonas cichorii, Pseudomonas marginalis),* bacterial soft rot (*Pectobacterium carotovorum*), downy mildew (*Bremia lactucae*)*,* gray mold *(Botrytis cinerea),* stem rot *(Sclerotinia sclerotiorum),* big-vein disease (*Mirafiori lettuce big-vein ophiovirus*), root rot (*Fusarium oxysporum*), bottom rot (*Rhizoctonia solani*), powdery mildew (*Golovinomyces orontii*), etc.

Green bean: stem rot *(Sclerotinia sclerotiorum),* gray mold *(Botrytis cinerea),* anthracnose *(Colletotrichum lindemuthianum*), angular leaf spot (*Phaeoisariopsis griseola*), etc.

Pea: Mycosphaerella blight (*Mycosphaerella blight*), gray mold *(Botrytis cinerea),* stem rot (*Sclerotinia sclerotiorum*), powdery mildew (*Erysiphe pisi),* etc.

Apple: powdery mildew (*Podosphaera leucotricha*), scab (*Venturia inaequalis*), Monilia leaf blight (*Monilinia mali*)*,* fruit spot (*Mycosphaerella pomi*), Valsa canker (*Valsa mali*)*,* Alternaria blotch (*Alternaria mali*)*,* rust (*Gymnosporangium yamadae*)*,* ring rot (*Botryosphaeria berengeriana*)*,* bitter rot (*Glomerella cingulata, Colletotrichum acutatum),* blotch (*Diplocarpon mali*)*,* fly speck (*Zygophiala jamaicensis*), sooty blotch (*Gloeodes pomigena*), violet root rot (*Helicobasidium mompa*), Rosellinia root rot (*Rosellinia necatrix*), gray mold *(Botrytis cinerea),* fire blight (*Erwinia amylovora*), silver leaf (*Chondrostereum purpureum*), crown gall (*Rhizobium radiobacter, Rhizobium rhizogenes*), etc.

Japanese apricot: scab (*Cladosporium carpophilum*)*,* gray mold (*Botrytis cinerea*), brown rot (*Monilinia mumecola*)*,* sooty blotch (*Peltaster* sp.), pocket (*Taphrina pruni*)*,* brown shot hole (*Phloeosporella padi*), etc.

Japanese persimmon: powdery mildew (*Phyllactinia kakicola*), bitter rot (*Gloeosporium kaki*), angular leaf spot *(Cercospora kaki*), circular leaf spot *(Mycosphaerella nawae*), gray mold (*Botrytis cinerea),* fly speck (*Zygophiala jamaicensis*), etc.

Peach: brown rot (*Monilinia fructicola, Monilia fructigena*), scab (*Cladosporium carpophilum),* Phomopsis rot (*Phomopsis* sp.) bacterial leaf spot (*Xanthomonas campesrtis* pv. *pruni*), leaf curl (*Taphrina deformans*), anthracnose (*Colletotrichum gloeosporioides*), Cylindrosporium leaf spot (*Phloeosporella padi*), Coriolus stem rot (*Coriolus versicolor*), etc.

Almond: brown rot (*Monilinia laxa*), leaf spot (*Stigmina carpophila*), scab (*Cladosporium carpophilum*), leaf blister (*Polystigma rubrum*), Alternaria blotch (*Alternaria alternate),* anthracnose *(Colletotrichum gloeosporioides*), etc.

Sweet cherry: brown rot (*Monilinia fructicola*), anthracnose (*Colletotrichum acutatum*), black spot (*Alternaria solani),* young-fruit rot (*Monilinia kusanoi*), Cylindrosporium leaf spot (*Mycosphaerella cerasella*), powdery mildew (*Podosphaera tridactyla),* etc.

Grape: gray mold *(Botrytis cinerea),* powdery mildew (*Uncinula necator*), late rot (*Glomerella cingulate, Colletotrichum acutatum),* downy mildew (*Plasmopara viticola*), anthracnose (*Elsinoe ampelina),* leaf blight (*Pseudocercospora vitis*), black rot (*Guignardia bidwellii),* white rot *(Coniella castaneicola*), rust *(Phakopsora ampelopsidis),* cottony bunch (pathogen unidentified), crown gall (*Rhizobium radiobacter, Rhizobium vitis*), etc.

Pear: scab (*Venturia nashicola),* rust (*Gymnosporangium asiaticum),* black spot *(Alternaria kikuchiana*), ring rot (*Botryosphaeria berengeriana*), powdery mildew (*Phyllactinia mali),* Phomopsis canker *(Phomopsis fukushii*), brown spot (*Stemphylium vesicarium)*,bitter rot *(Glomerella cingulata),* etc.

Tea plant: gray blight *(Pestalotiopsis longiseta, P. theae*), anthracnose (*Colletotrichum theae-sinensis),* net blister blight (*Exobasidium reticulatum*), bacterial shoot blight (*Pseudomonas syringae*), blister blight (*Exobasidium vexans*), etc.

Citrus: scab (*Elsinoe fawcettii),* blue mold (*Penicillium italicum),* green mold (*Penicillium digitatum),* gray mold (*Botrytis cinerea),* black spot (*Diaporthe citri),* canker (*Xanthomonas campestris* pv. *Citri),* powdery mildew *(Oidium* sp.), Phytophthora rot (*Phytophthora citrophthora),* anthracnose *(Colletotrichum fioriniae),* etc.

Kiwifruit: bacterial blossom blight *(Pseudomonas marginalis, Pseudomonas syringae, Pseudomonas viridiflava*), bacterial canker *(Pseudomonas syringae*), gray mold (*Botrytis cinerea),* fruit soft rot (*Botryosphaeria dothidea, Diaporthe* sp., *Lasiodiplodia theobromae),* sooty spot *(Pseudocercospora actinidiae),* etc.

Olive: anthracnose (*Colletotrichum acutatum, Colletotrichum gloeosporioides*), peacock spot (*Spilocaea oleaginea*), etc.

Japanese chestnut: anthracnose *(Colletotrichum gloeosporioides*), etc.

Wheat: powdery mildew (*Blumeria graminis* f.sp. *tritici*), red mold (*Gibberella zeae, Fusarium avenaceum, Fusarium culmorum, Fusarium crookwellense, Microdochium nivale*), red rust (*Puccinia recondita*), yellow rust (*Puccinia striiformis*), browning root rot *(Pythium iwayamai*), snow mold (*Monographella nivalis*), eyespot *(Pseudocercosporella herpotrichoides),* speckled leaf blotch (*Septoria tritici*), glume blotch (*Leptosphaeria nodorum),* Typhula snow blight (*Typhula incarnata*), Sclerotinia snow blight (*Myriosclerotinia borealis*), Take-all (*Gaeumannomyces graminis*), ergot (*Claviceps purpurea*), bunt (*Tilletia caries*), loose smut (*Ustilago nuda*), blast (*Pyricularia grisea*), damping off *(Pythium* spp., *Fusarium* spp., *Rhizoctonia* spp.), seedling blight *(Pythium* spp., *Fusarium* spp., *Rhizoctonia* spp.), etc.

Barley: stripe (*Pyrenophora graminea*), net blotch (*Pyrenophora teres*), scald, (*Rhynchosporium secalis*), loose smut (*Ustilago tritici, U.nuda*), damping off *(Pythium* spp., *Fusarium* spp., *Rhizoctonia* spp.), seedling blight *(Pythium* spp., *Fusarium* spp., *Rhizoctonia* spp.), etc.

Rice: blast (*Pyricularia oryzae*), sheath blight (*Rhizoctonia solani*), "Bakanae" disease (*Gibberella fujikuroi*), brown spot (*Cochliobolus miyabeanus*), seedling blight (*Pythiumgraminicola*), bacterial leaf blight (*Xanthomonas oryzae*), bacterial seedling blight (*Burkholderia plantarii*), bacterial brown stripe (*Acidovorax avenae*), bacterial grain rot (*Burkholderia glumae*), Cercospora leaf spot (*Cercospora oryzae*), false smut (*Ustilaginoidea virens*), discoloured rice grains (*Alternaria alternate, Curvularia intermedia*), kernel discoloration (*Alternaria padwickii*), pink coloring of rice grains (*Epicoccum purpurascens*), etc.

Tobacco: stem rot (*Sclerotinia sclerotiorum*), powdery mildew (*Erysiphe cichoracearum*), black shank (*Phytophthora nicotianae*), etc.

Tulip: gray mold (*Botrytis cinerea),* Botrytis blight (*Botrytis tulipae*), leaf rot (*Rhizoctonia solani*), bulb rot (*Fusarium oxysporum*), bulb-coat rot (*Rhizoctonia solani*), etc.

Rose: scab (*Diplocarpon mali*), powdery mildew (*Erysiphe simulans, Podosphaera pannosa*), gray mold (*Botrytis cinerea*), etc.

Chrysanthemum: gray mold (*Botrytis cinerea*), white rust (*Puccinia horiana*), downy mildew (*Paraperonospora minor, Peronospora danica*), Pythium blight (*Pythium aphanidermatum, Pythium dissotocum, Pythium helicoides, Pythium oedochilum, Pythium sylvaticum*), root and stem rot (*Rhizoctonia solani*), Fusarium blight (*Fusarium solani*), etc.

Gerbera: gray mold (*Botrytis cinerea),* powdery mildew (*Podosphaera xanthii*), etc.

Lily: Botrytis blight (*Botrytis elliptica, Pestalotiopsis* sp.), gray mold (*Botrytis cinerea*), etc.

Sunflower: downy mildew (*Plasmopara halstedii*), stem rot (*Sclerotinia sclerotiorum*), gray mold (*Botrytis cinerea*), etc.

Bentgrass: Sclerotinia snow blight (*Sclerotinia borealis*), large patch (*Rhizoctonia solani*), brown patch (*Rhizoctonia solani*), dollar spot (*Sclerotinia homoeocarpa*), blast (*Pyricularia* sp.), Pythium red blight (*Pythium aphanidermatum*), anthracnose (Colletotrichum graminicola), etc.

Orchard grass: powdery mildew (*Erysiphe graminis*), etc.

Soybean: purple stain (*Cercospora kikuchii*), downy mildew (*Peronospora manshurica*), Phytophthora rot (*Phytophthora sojae*), rust (*Phakopsora pachyrhizi*), stem rot (*Sclerotinia sclerotiorum*), anthracnose (*Colletotrichum truncation*), gray mold (*Botrytis cinerea*), anthracnose (*Elsinoe glycines*), pod and stem blight (*Diaporthe phaseolorum* var. *sojae*), damping off (*Pythium* spp., *Fusarium* spp., *Rhizoctonia* spp.), seedling blight (*Pythium* spp., *Fusarium* spp., *Rhizoctonia* spp.), etc.

Potato: late blight (*Phytophthora infestans*), early blight (*Alternaria solani*), black scurf (*Thanatephorus cucumeris*), Verticillium wilt (*Verticillium albo-atrum, V. dahliae, V. nigrescens*), black leg (*Pectobacterium atrosepticum*), bacterial soft rot (*Pectobacterium carotovorum*), gray mold (*Botrytis cinerea*), scab (*Streptomyces* spp.), stem rot (*Sclerotinia sclerotiorum*), etc.

Japanese yam: leaf rust (*Cylindrosporium dioscoreae*), anthracnose (*Colletotrichum gloeosporioides*), blue mold (*Penicillium sclerotigenum*), etc.

Sweet potato: violet root rot (*Helicobasidium mompa*), vine split blight (*Fusarium oxysporum*), etc.

Taro: Phytophthora blight (*Phytophthora colocasiae*), bulb-coat rot (*Rhizoctonia solani*), etc.

Ginger: Pythium rot (*Pythium ultimum, Pythium myriotylum*), leaf spot (*Phyllosticta zingiberis*), etc.

Banana: Fusarium wilt (*Fusarium oxysporum*), Sigatoka disease (*Mycosphaerella fijiensis, M. musicola*), etc.

Mango: anthracnose (*Colletotrichum aenigma*), bacterial canker (*Xanthomonas campestris*), stem-end rot (*Diaporthe pseudophoenicicola, Lasiodiplodia theobromae, Lasiodiplodia* spp., *Neofusicoccum parvum, Neofusicoccum* sp.), gray mold (*Botrytis cinerea*), etc.

Rapeseed: stem rot (*Sclerotinia sclerotiorum*), black leg (*Phoma lingam*), black spot (*Alternaria brassicae*), powdery mildew (*Erysiphe cruciferarum, Erysiphe cichoracearum, Oidium matthiolae*), downy mildew (*Peronospora parasitica*), etc.

Coffee: rust (*Hemileia vastatrix*), anthracnose (*Colletotrichum coffeanum*), leaf spot (*Cercospora coffeicola*), etc.

Sugarcane: brown rust (*Puccinia melanocephala*), etc.

Cone: zonate leaf spot (*Gloeocercospora sorghi*), rust (*Puccinia sorghi*), southern rust (*Puccinia polysora*), smut (*Ustilago maydis*), southern leaf blight (*Cochliobolus heterostrophus*), northern leaf blight (*Setosphaeria turcica*), damping off (*Pythium* spp., *Fusarium* spp., *Rhizoctonia* spp.), seedling blight (*Pythium* spp., *Fusarium* spp., *Rhizoctonia* spp.), etc.

Cotton plant: seedling blight (*Pythium* sp.), rust (*Phakopsora gossypii*), frosty mildew (*Mycosphaerella areola*), anthracnose (*Glomerella gossypii*), etc.

Hop: downy mildew (*Pseudoperonospora humuli*), powdery mildew (*Oidium* sp., *Podosphaera macularis*), gray mold (*Botrytis cinerea*), etc.

The agricultural/horticultural fungicidal composition of the present invention may be applied to various parts of plants, such as leaves, stems, stalks, flowers, buds, fruits, seeds, sprouts, roots, tubers, tuberous roots, seedlings, and/or cuttings. In addition, improved varieties, cultivars, mutants, hybrids, and genetically modified organisms (GMOs) of these plants are also applicable.

The agricultural/horticultural fungicidal composition of the present invention may be used for seed treatment, foliar spray, soil application, and water application to control various diseases occurring on agricultural/horticultural crops including flowers, turf, or pasture grasses.

The agricultural/horticultural fungicidal composition of the present invention may be used as a seed treatment agent, and any form of seed treatment known to those skilled in the art may be used, including soaking seeds in the composition, submerging them, or coating them with the composition. The seed treatment should be conducted prior to seeding.

Applying to seeds this composition in the form of a coating is a preferred form, and seeds coated with a layer containing an agricultural/horticultural fungicidal composition of the present invention may also be used.

Regarding the coating, seeds may be coated using a variety of processes known in the art. For example, the coating process may include spraying a seed treatment composition onto the surfaces of seeds while agitating the seeds in suitable equipment such as a tumbler or pan-type granulator.

The agricultural/horticultural fungicidal composition of the present invention may contain an additional component(s) other than the component (I) and component (II). As the additional component(s), known ingredients used for formulation may be exemplified.

As the dosage form of the agricultural/horticultural fungicidal composition of the present invention, a common agrochemical form, that is, as the dosage form, dustable powder (DP), wattable powder (WP), emulsifiable concentrate

(EC), flowable (FL), suspension concentrate (SC), water soluble powder (SP), water dispersible granules (WG), tablets, granules (GR), suspo emulsion (SE), oil dispersion (OD), or emulsion-oil-in-water (EW) may be exemplified. Formulation is not limited by any particular technique or procedure, but may be performed by any known technique or procedure.

In an aspect of the present invention, auxiliary preparation materials used in the formulation such as various carriers, solvents, or additives are not particularly limited. One aspect of the present invention is a fungicidal composition containing an agrochemically acceptable solid and/or liquid carrier.

In the case of using a solid dosage form, the solid carrier may be used, including plant powder (e.g., soybean flour, wheat flour), mineral fine powder (e.g., diatomaceous earth, phospholite, gypsum, talc, bentonite, pyrophyllite, clay), or an organic/inorganic compound (e.g., sodium benzoate, urea, mirabilite).

In the case of using a liquid dosage form, the liquid carrier may be used, including petroleum distillate (e.g., kerosene, xylene, solvent naphtha), cyclohexane, cyclohexanone, dimethylformamide, dimethyl sulfoxide, alcohol, acetone, trichloroethylene, methyl isobutyl ketone, mineral oil, vegetable oil, or water.

For the formulation, a surfactant may be optionally added. As the surfactant, a nonionic surfactant (e.g., a polyoxyethylene adduct of alkyl phenyl ether, a polyoxyethylene adduct of alkyl ether, a polyoxyethylene adduct of higher fatty acid ester, a polyoxyethylene adduct of sorbitan higher fatty acid ester, a polyoxyethylene adduct of tristyrylphenyl ether), polyoxyethylene-added alkyl phenyl ether sulfate, or a formaldehyde condensate of alkylbenzenesulfonate, higher alcohol sulfate, alkyl naphthalene sulfonate, polycarboxylate, lignin sulfonate, or alkylnaphthalene sulfonate, or an isobutylene-maleic anhydride copolymer may be exemplified.

The concentration of active ingredients in the fungicidal composition of the present invention may be set, if appropriate, according to the dosage form. For example, the concentration of active ingredients in a wattable powder is preferably from 5 to 90 wt% and more preferably from 10 to 85 wt%. The concentration of active ingredients in an emulsion is preferably from 3 to 70 wt% and more preferably from 5 to 60 wt%. The concentration of active ingredients in granules is preferably from 0.01 to 50 wt% and more preferably from 0.05 to 40 wt%.

The method of preparing a preparation is not particularly limited, and any known preparation method may be employed depending on the dosage form.

The following shows some Preparation Examples. Note that the preparation formulation shown below is merely examples and may be modified without departing from the main purpose of the present invention. The present invention is not limited in any way by the following Preparation Examples. Unless otherwise specified, the "parts" means "parts by weight."

(Preparation Example 1: Wattable Powder)

40 parts of compounds of components (I) and (II), 53 parts of diatomaceous earth, 4 parts of higher alcohol sulfate, and 3 parts of alkylnaphthalene sulfonate are homogenously mixed and finely pulverized to give wattable powder with 40% active ingredients.

(Preparation Example 2: Emulsifiable Concentrate)

30 parts of compounds of components (I) and (II), 33 parts of xylene, 30 parts of dimethylformamide, and 7 parts of polyoxyethylene alkylallyl ether are mixed and dissolved to give an emulsifiable concentrate with 30% active ingredients.

(Preparation Example 3: Granules)

5 parts of compounds of components (I) and (II), 40 parts of talc, 38 parts of clay, 10 parts of bentonite, and 7 parts of sodium alkyl sulfate are homogenously mixed, finely pulverized, and then granulated to a particle diameter of 0.5 to 1.0 mm to give granules with 5% active ingredients.

(Preparation Example 4: Granules)

5 parts of compounds of components (I) and (II), 73 parts of clay, 20 parts of bentonite, 1 part of dioctyl sodium sulfosuccinate, and 1 part of potassium phosphate were homogenously mixed and pulverized. Water is added and the mixture is kneaded well, then granulated and dried to give granules with 5% active ingredients.

(Preparation Example 5: Suspension Concentrate)

10 parts of compounds of components (I) and (II), 4 parts of polyoxyethylene alkylallyl ether, 2 parts of sodium polycarboxylate, 10 parts of glycerin, 0.2 parts of xanthan gum, and 73.8 parts of water are mixed and wet-pulverized

until the particle size is 3 microns or less to give a suspension concentrate with 10% active ingredients.

(Preparation Example 6: Water Dispersible Granules)

40 parts of compounds of components (I) and (II), 36 parts of clay, 10 parts of potassium chloride, 1 part of sodium alkyl benzene sulfonate, 8 parts of sodium lignin sulfonate, and 5 parts of formaldehyde condensate of sodium alkyl benzene sulfonate are homogenously mixed and finely pulverized. Next, an appropriate amount of water is added, and the mixture is kneaded to form a clay. The clay-like material is granulated and then dried to give water dispersible granules with 40% active ingredients.

The fungicidal effects exerted by each agricultural/horticultural fungicidal composition of the present invention are demonstrated in the following Test Examples.

The compounds with compound Nos. 1 to 12 in component (I) of the tables are the same as the compounds with compound Nos. 1 to 12 in Table 1 above.

(Test Example) Fungicidal Test for Wheat Leaf Blight

Compounds of component (I) and component (II) were dissolved in dimethyl sulfoxide at 10,000 ppm and diluted with water to the prescribed concentration to prepare a chemical agent solution. A mixture of the prescribed concentration of the chemical agent solution, wheat leaf blight pathogen (*Septoria tritici*) conidia, and malt extract was prepared on a microplate and incubated at 20°C in the dark for 3 days. The turbidity at a wavelength of 405 nm was measured with a microplate reader, and the growth of wheat leaf blight pathogen was compared with that of no treatment control to determine the growth inhibition rate. The results are shown in Tables 2 to 7, along with the expected values calculated as below.

Each expected value was calculated from the fungicidal efficacy of a single application of the compound of component (I) and the fungicidal efficacy of a single application of compound of component (II) by using Colby's equation (S.R. Colby, Calculating synergistic and antagonistic responses of herbicide combinations, Weeds, 15, 20-22 pp (1967)).

$$E = M + N - (M \times N)/100$$

where E represents the expected value of the growth inhibition rate (%) for the composition containing component (I) and component (II), M represents the growth inhibition rate (%) when the component (I) is used alone, and N represents the growth inhibition rate (%) when the component (II) is used alone. When the measured growth inhibition rate (%) for the composition containing component (I) and component (II) is greater than the expected value E, the composition containing components(I) and component (II) should exert a synergistic effect.

In Tables 2 to 7, some favorable synergistic effects were observed for all combinations of components (I) and (II) .

[Table 2]

Result of wheat leaf blight fungicidal test (growth inhibition rate) - in the case where component (I) is Compound 1

| Component (II) \ Component (I): Compound 1 | | 100ppm | 50ppm | 25ppm | 12.5ppm | 6.3ppm | None |
|---|---|---|---|---|---|---|---|
| Azoxystrobin | 1ppm | | | 100 (73) | | | 10 |
| Ipflufenoquin | 0.25ppm | | | | 97 (37) | | 6 |
| Inpyrfluxam | 0.01ppm | | | | 84 (68) | | 24 |
| Epoxiconazole | 0.02ppm | | 100 (87) | | | | 27 |
| Chlorothalonil | 0.001ppm | | 95 (84) | | | | 8 |
| Cyazofamid | 64ppm | | 100 (87) | | | | 27 |
| Difenoconazole | 4ppm | | | | | 100 (92) | 85 |
| Cyproconazole | 1ppm | | | | 92 (58) | | 1 |
| Tebuconazole | 0.063ppm | 100 (93) | | | | | 33 |
| Triflumizole | 0.06ppm | | | | | 79 (50) | 12 |
| Trifloxystrobin | 0.25ppm | | | | | 89 (55) | 21 |
| Bixafen | 0.004ppm | | | | 93 (69) | | 27 |
| Picoxystrobin | 0.02ppm | | | | 82 (59) | | 2 |
| Pyraclostrobin | 0.02ppm | | | | | 80 (57) | 24 |
| Fluazinam | 0.004ppm | | | 100 (70) | | | 0 |
| Fluxapyroxad | 0.001ppm | | | 100 (80) | | | 32 |
| Prothioconazole | 0.02ppm | | | 100 (73) | | | 10 |
| Florylpicoxamid | 0002ppm | | | | 94 (87) | | 70 |
| Benzovindiflupyr | 0.04ppm | | | | | 98 (80) | 65 |
| Mancozeb | 0.06ppm | | | | 93 (66) | | 19 |
| Copper sulfate | 0.25ppm | | | 100 (72) | | | 8 |
| None | | 90 | 82 | 70 | 58 | 44 | |

Each value in the table: growth inhibition rate (%); (): expected value calculated by Colby's equation.

[Table 3]

Result of wheat leaf blight fungicidal test (growth inhibition rate) - in the case where component (I) is Compound 2

| Component (II) \ Component (I): Compound 2 | | 100ppm | 50ppm | 25ppm | 12.5ppm | 6.3ppm | None |
|---|---|---|---|---|---|---|---|
| Azoxystrobin | 1ppm | | | | 79 (52) | | 10 |
| Ipflufenoquin | 1ppm | | | 100 (63) | | | 22 |
| Inpyrfluxam | 0.01ppm | | | 82 (64) | | | 24 |
| Epoxiconazole | 0.02ppm | | 81 (70) | | | | 27 |
| Chlorothalonil | 0.04ppm | | 76 (63) | | | | 9 |
| Cyazofamid | 64ppm | 100 (73) | | | | | 27 |
| Difenoconazole | 16ppm | | 100 (93) | | | | 83 |
| Cyproconazole | 1ppm | | | 76 (52) | | | 1 |
| Tebuconazole | 0.016ppm | | | | 60 (46) | | 0 |
| Triflumizole | 0.25ppm | | | | 87 (52) | | 11 |
| Trifloxystrobin | 0.25ppm | | | | | 99 (48) | 21 |
| Bixafen | 0.004ppm | | | | 91 (61) | | 27 |
| Picoxystrobin | 0.06ppm | | 94 (60) | | | | 3 |
| Pyraclostrobin | 0.02ppm | | | | 93 (59) | | 24 |
| Fluazinam | 0.004ppm | | | 75 (52) | | | 0 |
| Fluxapyroxad | 0.001ppm | | | | 92 (63) | | 32 |
| Prothioconazole | 1ppm | | | | 100 (51) | | 8 |
| Florylpicoxamid | .0002ppm | | | 96 (86) | | | 70 |
| Benzovindiflupyr | 0.04ppm | | | | | 99 (77) | 65 |
| Mancozeb | 0.02ppm | | | 89 (61) | | | 19 |
| Copper sulfate | 1ppm | | | 80 (64) | | | 24 |
| None | | 62 | 59 | 52 | 46 | 34 | |

Each value in the table: growth inhibition rate (%); (): expected value calculated by Colby's equation.

[Table 4]

Result of wheat leaf blight fungicidal test (growth inhibition rate) - in the case where component (I) is Compound 3

| Component (II) \ Component (I): Compound 3 | | 100ppm | 50ppm | 25ppm | 12.5ppm | 6.3ppm | None |
|---|---|---|---|---|---|---|---|
| Azoxystrobin | 1ppm | | 69 (50) | | | | 10 |
| Ipflufenoquin | 1ppm | | | 72 (55) | | | 22 |
| Inpyrfluxam | 0.04ppm | | | | 100 (70) | | 49 |
| Epoxiconazole | 64ppm | 100 (97) | | | | | 94 |
| Chlorothalonil | 0.02ppm | | | 62 (43) | | | 2 |
| Cyazofamid | 16ppm | | | | 79 (43) | | 4 |
| Difenoconazole | 0.04ppm | | 94 (86) | | | | 75 |
| Cyproconazole | 0.25ppm | | | | | 73 (42) | 15 |
| Tebuconazole | 0.016ppm | | | 44 (42) | | | 0 |
| Triflumizole | 0.25ppm | | | | 89 (47) | | 11 |
| Trifloxystrobin | 0.25ppm | | | | | 95 (46) | 21 |
| Bixafen | 0.004ppm | | | | 95 (65) | | 27 |
| Picoxystrobin | 0.02ppm | | | 71 (43) | | | 2 |
| Pyraclostrobin | 0.02ppm | | | 72 (56) | | | 24 |
| Fluazinam | 0.004ppm | | 76 (44) | | | | 0 |
| Fluxapyroxad | 0.001ppm | | | | 87 (60) | | 32 |
| Prothioconazole | 0.02ppm | | 78 (50) | | | | 10 |
| Florylpicoxamid | 0.001ppm | | | | 99 (88) | | 80 |
| Benzovindiflupyr | 0.01ppm | | | | | 94 (65) | 48 |
| Mancozeb | 0.06ppm | | 67 (55) | | | | 19 |
| Copper sulfate | 1ppm | 93 (61) | | | | | 24 |
| None | | 49 | 44 | 42 | 41 | 32 | |

Each value in the table: growth inhibition rate (%); (): expected value calculated by Colby's equation.

[Table 5]

Result of wheat leaf blight fungicidal test (growth inhibition rate)-in the case where component (I) is Compound 4

| Component (II) \ Component (I): Compound 4 | | 100ppm | 50ppm | 25ppm | 12.5ppm | 6.3ppm | None |
|---|---|---|---|---|---|---|---|
| Azoxystrobin | 1ppm | | | | 74 (54) | | 10 |
| Ipflufenoquin | 1ppm | | | | 100 (60) | | 22 |
| Inpyrfluxam | 0.04ppm | | | | 99 (74) | | 49 |
| Epoxiconazole | 0.02ppm | | | 100 (81) | | | 27 |
| Chlorothalonil | 0.02ppm | | | | | 60 (37) | 2 |
| Cyazofamid | 64ppm | | | | 91 (63) | | 27 |
| Difenoconazole | .0002ppm | | | 100 (92) | | | 69 |
| Cyproconazole | 1ppm | | | 87 (75) | | | 1 |
| Tebuconazole | 0.016ppm | | | 100 (75) | | | 0 |
| Triflumizole | 0.25ppm | | | | 100 (54) | | 11 |
| Trifloxystrobin | 0.25ppm | | | | | 100 (49) | 21 |
| Bixafen | 0.004ppm | | | | | 99 (67) | 27 |
| Picoxystrobin | 1ppm | | | 93 (76) | | | 8 |
| Pyraclostrobin | 0.02ppm | | | 100 (81) | | | 24 |
| Fluazinam | 0.004ppm | | | | 98 (49) | | 0 |
| Fluxapyroxad | 0.001ppm | | | | | 93 (57) | 32 |
| Prothioconazole | 1ppm | | | | 100 (53) | | 8 |
| Florylpicoxamid | 0.001ppm | | | | 100 (90) | | 80 |
| Benzovindiflupyr | 0.01ppm | | | | | 90 (67) | 48 |
| Mancozeb | 0.06ppm | | | | 83 (59) | | 19 |
| Copper sulfate | 1ppm | | | 100 (81) | | | 24 |
| None | | 100 | 99 | 75 | 49 | 36 | |

Each value in the table: growth inhibition rate (%); (): expected value calculated by Colby's equation.

[Table 6]

Result of wheat leaf blight fungicidal test (growth inhibition rate)-in the case where component (I) is Compound 5

| Component (I): Compound 5 / Component (II) | | 100ppm | 50ppm | 25ppm | 12.5ppm | 6.3ppm | None |
|---|---|---|---|---|---|---|---|
| Azoxystrobin | 0.06ppm | | 78 (68) | | | | 10 |
| Ipflufenoquin | 0.25ppm | 91 (74) | | | | | 6 |
| Inpyrfluxam | 0.01ppm | | | | 100 (76) | | 24 |
| Epoxiconazole | 0.02ppm | | | | | 89 (71) | 27 |
| Chlorothalonil | 0.04ppm | | 93 (68) | | | | 9 |
| Cyazofamid | 0.25ppm | 82 (72) | | | | | 0 |
| Difenoconazole | .0002ppm | 99 (91) | | | | | 69 |
| Cyproconazole | 0.25ppm | | | 86 (73) | | | 15 |
| Tebuconazole | 64ppm | | 99 (91) | | | | 76 |
| Triflumizole | 0.25ppm | | | | | 87 (66) | 11 |
| Trifloxystrobin | 0.25ppm | | | 93 (75) | | | 21 |
| Bixafen | 0.004ppm | | | | 94 (76) | | 27 |
| Picoxystrobin | 0.02ppm | | | | | 71 (62) | 2 |
| Pyraclostrobin | 0.25ppm | | | 100 (96) | | | 86 |
| Fluazinam | 0.004ppm | | | 86 (68) | | | 0 |
| Fluxapyroxad | 1ppm | | | | | 100 (94) | 86 |
| Prothioconazole | 0.25ppm | 91 (74) | | | | | 7 |
| Florylpicoxamid | 0.001ppm | | | | | 100 (92) | 80 |
| Benzovindiflupyr | 0.04ppm | | | | 100 (89) | | 65 |
| Mancozeb | 0.06ppm | | 96 (71) | | | | 19 |
| Copper sulfate | 1ppm | | | 87 (76) | | | 24 |
| None | | 72 | 64 | 68 | 68 | 61 | |

Each value in the table: growth inhibition rate (%); (): expected value calculated by Colby's equation.

[Table 7]

Result of wheat leaf blight fungicidal test (growth inhibition rate)-in the case where component (I) is Compound 6

| Component (II) \ Component (I): Compound 6 | 100ppm | 50ppm | 25ppm | 12.5ppm | 6.3ppm | None |
|---|---|---|---|---|---|---|
| Azoxystrobin 1ppm | | | | | 67 (52) | 10 |
| Ipflufenoquin 0.06ppm | 91 (71) | | | | | 6 |
| Inpyrfluxam 0.01ppm | | | | | 87 (60) | 24 |
| Epoxiconazole 64ppm | | | | 100 (97) | | 94 |
| Chlorothalonil 0.02ppm | | | | | 77 (48) | 2 |
| Cyazofamid 4ppm | | | | | 74 (50) | 6 |
| Difenoconazole 0.004ppm | 99 (92) | | | | | 75 |
| Cyproconazole 1ppm | | | | | 58 (47) | 1 |
| Tebuconazole 64ppm | | | | | 93 (87) | 76 |
| Triflumizole 0.06ppm | | | | | 71 (53) | 12 |
| Trifloxystrobin 0.25ppm | 95 (76) | | | | | 21 |
| Bixafen 0.004ppm | | | | | 97 (61) | 27 |
| Picoxystrobin 0.25ppm | 80 (72) | | | | | 8 |
| Pyraclostrobin 0.02ppm | 93 (77) | | | | | 24 |
| Fluazinam 0.004ppm | 93 (69) | | | | | 0 |
| Fluxapyroxad 0.02ppm | | | 98 (86) | | | 63 |
| Prothioconazole 0.25ppm | 95 (71) | | | | | 7 |
| Florylpicoxamid 0.001ppm | | | | | 99 (89) | 80 |
| Benzovindiflupyr 0.04ppm | | | | 100 (85) | | 65 |
| Mancozeb 0.004ppm | | | | | 79 (57) | 9 |
| Copper sulfate 16ppm | | 100 (90) | | | | 73 |
| None | 69 | 64 | 63 | 58 | 47 | |

Each value in the table: growth inhibition rate (%); (): expected value calculated by Colby's equation.

(Test Example) Soybean Rust Control Test

Compounds of component (I) and component (II) were dissolved in dimethyl sulfoxide and diluted with water to the prescribed concentration to prepare a chemical agent solution. A mixture of the prescribed concentration of the chemical agent solution and sterile water was prepared on a microplate, and leaf discs cut from soybean leaves were floated on the microplate. Uredospores of soybean rust pathogen (*Phakopsora pachyrhizi*) were inoculated and incubated at 25°C for 9 days. The area percentage of lesions was determined by observing uredospore-forming lesions on the leaf discs, and the control value was calculated. The control value was calculated by the following formula:

Control value (%) = 100 - {Area percentage of lesions in treatment plot/Area percentage of lesions in untreatment plot} × 100.

The results are shown in Tables 8 to 18, along with the expected values calculated as below. Each expected values was calculated from the fungicidal efficacy of a single application of compound of component (I) and the fungicidal efficacy of a single application of compound of component (II) by using Colby's equation:

$$E = M + N - (M \times N)/100$$

where E represents the expected value of the control value for the composition containing component (I) and component (II), M represents the control value (%) when the component (I) is used alone, and N represents the control value (%)

when the component (II) is used alone. When the measured control value for the composition containing component (I) and component (II) is greater than the expected value E, the composition containing component (I) and composition (II) should exert a synergistic effect.

In Tables 8 to 18, some favorable synergistic effect was observed for all combinations of component (I) and component (II).

[Table 8]

Result of soybean rust control test (control value)-in the case where component (I) is Compound 1

| Component (II) \ Component (I): Compound 1 | | 0.4 ppm | 0.1 ppm | 0.025 ppm | 0.0063 ppm | None |
|---|---|---|---|---|---|---|
| Azoxystrobin | 0.0001 ppm | 100 *99* | 100 *75* | 50 *48* | 50 *12* | 0 |
| Inpyrfluxam | 0.000025 ppm | 100 *99* | | | | 0 |
| Chlorothalonil | 0.0063 ppm | 100 *99* | | | 50 *12* | 0 |
| Difenoconazole | 0.000025 ppm | 100 *99* | 100 *75* | 50 *48* | | 0 |
| Cyproconazole | 0.000025 ppm | 100 *99* | 83 *75* | 67 *48* | 33 *12* | 0 |
| Tebuconazole | 0.000025 ppm | 100 *99* | 100 *81* | 75 *61* | 75 *34* | 25 |
| Trifloxystrobin | 0.0004 ppm | 100 *99* | 100 *87* | 100 *74* | 100 *56* | 50 |
| Trifloxystrobin | 0.0001 ppm | 100 *99* | 100 *75* | 50 *48* | | 0 |
| Pyraclostrobin | 0.0001 ppm | 100 *99* | | | | 50 |
| Fluazinam | 0.0063 ppm | 100 *99* | | | | 33 |
| Fluxapyroxad | 0.000025 ppm | 100 *99* | | | 33 *12* | 0 |
| Prothioconazole | 0.000025 ppm | 100 *99* | 100 *75* | 100 *48* | | 0 |
| Florylpicoxamid | 0.0001 ppm | | 88 *81* | | | 25 |
| Benzovindiflupyr | 0.000025 ppm | 100 *99* | 83 *83* | 67 *65* | | 33 |
| Mancozeb | 0.0063 ppm | 100 *99* | | 50 *48* | | 0 |
| None | | 99 | 75 | 48 | 12 | |

Each value in the table: (left) control value, (right italics) expected value calculated by Colby's equation.

[Table 9]

Result of soybean rust control test (control value)-in the case where component (I) is Compound 3

| Component (I): Compound 3 / Component (II) | | 0.4 ppm | 0.1 ppm | 0.025 ppm | 0.0063 ppm | None |
|---|---|---|---|---|---|---|
| Azoxystrobin | 0.0001 ppm | 100 *93* | | | | 50 |
| Inpyrfluxam | 0.000025 ppm | 100 *90* | | | | 33 |
| Chlorothalonil | 0.0063 ppm | 100 *90* | | | | 33 |
| Difenoconazole | 0.000025 ppm | 100 *89* | | | | 25 |
| Cyproconazole | 0.000025 ppm | 100 *90* | | | | 33 |
| Tebuconazole | 0.000025 ppm | 100 *90* | 67 *57* | 67 *48* | 67 *41* | 33 |
| Trifloxystrobin | 0.0001 ppm | 100 *85* | 75 *35* | 75 *22* | 50 *12* | 0 |
| Pyraclostrobin | 0.0001 ppm | 100 *93* | 75 *68* | 75 *61* | | 50 |
| Fluazinam | 0.0063 ppm | 100 *85* | 50 *35* | | | 0 |
| Fluxapyroxad | 0.000025 ppm | 100 *85* | | | | 0 |
| Prothioconazole | 0.000025 ppm | 100 *93* | | | | 50 |
| Florylpicoxamid | 0.0001 ppm | 100 *93* | | | | 50 |
| Benzovindiflupyr | 0.000025 ppm | 100 *85* | 75 *35* | 50 *22* | 25 *12* | 0 |
| Mancozeb | 0.0063 ppm | 100 *90* | 33 *57* | 67 *48* | | 33 |
| None | | 85 | 35 | 22 | 12 | |

Each value in the table: (left) control value, (right italics) expected value calculated by Colby's equation.

[Table 10]

Result of soybean rust control test (control value)-in the case where component (I) is Compound 4

| Component (I): Compound 4 / Component (II) | | 0.4 ppm | 0.1 ppm | 0.025 ppm | 0.0063 ppm | None |
|---|---|---|---|---|---|---|
| Inpyrfluxam | 0.000025 ppm | 100 *98* | | 75 *64* | | 50 |
| Chlorothalonil | 0.0063 ppm | 100 *97* | | 50 *28* | | 0 |
| Difenoconazole | 0.000025 ppm | 100 *97* | 75 *67* | 50 *28* | 50 *18* | 0 |
| Cyproconazole | 0.000025 ppm | 100 *97* | | | | 0 |
| Tebuconazole | 0.000025 ppm | 100 *98* | | | | 50 |
| Trifloxystrobin | 0.0001 ppm | 100 *97* | | | | 0 |
| Pyraclostrobin | 0.0001 ppm | 100 *98* | 100 *84* | | | 50 |
| Fluazinam | 0.0063 ppm | 100 *98* | 88 *84* | 75 *64* | | 50 |
| Fluxapyroxad | 0.000025 ppm | 100 *99* | | | | 75 |
| Prothioconazole | 0.000025 ppm | 100 *97* | 100 *67* | 50 *28* | | 0 |
| Florylpicoxamid | 0.0001 ppm | 100 *98* | | | | 50 |
| Benzovindiflupyr | 0.000025 ppm | 100 *98* | 100 *84* | 75 *64* | | 50 |
| Mancozeb | 0.0063 ppm | 100 *97* | | 33 *28* | 33 *18* | 0 |
| None | | 97 | 67 | 28 | 18 | |

Each value in the table: (left) control value, (right italics) expected value calculated by Colby's equation.

[Table 11]

Result of soybean rust control test (control value)-in the case where component (I) is Compound 5

| Component (II) \ Component (I): Compound 5 | | 0.4 ppm | | 0.1 ppm | | 0.025 ppm | | 0.0063 ppm | | None |
|---|---|---|---|---|---|---|---|---|---|---|
| Azoxystrobin | 0.0001 ppm | 100 | *93* | 75 | *59* | 50 | *39* | | | 25 |
| Inpyrfluxam | 0.000025 ppm | 100 | *90* | | | 25 | *18* | 25 | *7* | 0 |
| Chlorothalonil | 0.0063 ppm | 100 | *90* | | | | | | | 0 |
| Difenoconazole | 0.000025 ppm | 100 | *90* | 67 | *46* | 33 | | | | 0 |
| Cyproconazole | 0.000025 ppm | 100 | *90* | 75 | *46* | 50 | | | | 0 |
| Tebuconazole | 0.000025 ppm | 100 | *93* | 75 | *59* | 50 | *39* | 50 | *30* | 25 |
| Trifloxystrobin | 0.0001 ppm | 100 | *95* | 75 | *73* | | | | | 50 |
| Pyraclostrobin | 0.0001 ppm | | | 88 | *59* | 50 | *39* | 50 | *30* | 25 |
| Fluazinam | 0.0063 ppm | 100 | *90* | 50 | *46* | 50 | *18* | | | 0 |
| Fluxapyroxad | 0.000025 ppm | 100 | *93* | 75 | *59* | 50 | *39* | | | 25 |
| Prothioconazole | 0.000025 ppm | 100 | *93* | 75 | *59* | 75 | *39* | 50 | *30* | 25 |
| Florylpicoxamid | 0.0001 ppm | 100 | *90* | 75 | *46* | 50 | *18* | 50 | *7* | 0 |
| Benzovindiflupyr | 0.000025 ppm | | | | | 33 | *18* | 33 | *7* | 0 |
| Mancozeb | 0.0063 ppm | 100 | *90* | 88 | *46* | 50 | *18* | 50 | *7* | 0 |
| None | | 90 | | 46 | | 18 | | 7 | | |

Each value in the table: (left) control value, (right italics) expected value calculated by Colby's equation.

[Table 12]

Result of soybean rust control test (control value)-in the case where component (I) is Compound 6

| Component (II) \ Component (I): Compound 6 | | 0.4 ppm | | 0.1 ppm | | 0.025 ppm | | 0.0063 ppm | | None |
|---|---|---|---|---|---|---|---|---|---|---|
| Azoxystrobin | 0.0001 ppm | 100 | *100* | | | | | | | 75 |
| Inpyrfluxam | 0.000025 ppm | 100 | *100* | 100 | *94* | | | | | 75 |
| Chlorothalonil | 0.0063 ppm | 100 | *98* | | | | | | | 0 |
| Difenoconazole | 0.000025 ppm | 100 | *98* | | | | | | | 0 |
| Cyproconazole | 0.000025 ppm | 100 | *99* | 100 | *82* | 88 | *62* | 75 | *40* | 25 |
| Tebuconazole | 0.000025 ppm | 100 | *99* | 100 | *84* | 83 | *66* | 67 | *46* | 33 |
| Trifloxystrobin | 0.0001 ppm | 100 | *98* | 100 | *76* | 50 | *49* | 50 | *19* | 0 |
| Pyraclostrobin | 0.0001 ppm | 100 | *99* | 100 | *88* | 100 | *75* | 75 | *60* | 50 |
| Fluazinam | 0.0063 ppm | 100 | *98* | 100 | *76* | 50 | *49* | | | 0 |
| Fluxapyroxad | 0.000025 ppm | 100 | *100* | | | | | | | 75 |
| Prothioconazole | 0.000025 ppm | 100 | *98* | | | 75 | *49* | | | 0 |
| Florylpicoxamid | 0.0001 ppm | 100 | *98* | | | | | | | 0 |
| Benzovindiflupyr | 0.000025 ppm | 100 | *99* | | | 75 | *75* | 75 | *60* | 50 |
| Mancozeb | 0.0063 ppm | 100 | *99* | 100 | *84* | 67 | *66* | | | 33 |
| None | | 98 | | 76 | | 49 | | 19 | | |

Each value in the table: (left) control value, (right italics) expected value calculated by Colby's equation.

[Table 13]

Result of soybean rust control test (control value)-in the case where component (I) is Compound 7

| Component (II) \ Component (I): Compound 7 | 0.4 ppm | 0.1 ppm | 0.025 ppm | 0.0063 ppm | None |
|---|---|---|---|---|---|
| Azoxystrobin 0.0001 ppm | 100 *97* | | | | 50 |
| Inpyrfluxam 0.000025 ppm | 100 *96* | 75 *73* | 75 *49* | | 25 |
| Chlorothalonil 0.0063 ppm | 100 *94* | | 50 *32* | | 0 |
| Difenoconazole 0.000025 ppm | 100 *94* | 100 *64* | | | 0 |
| Cyproconazole 0.0001 ppm | 100 *99* | 100 *95* | 100 *92* | 100 *89* | 88 |
| Cyproconazole 0.000025 ppm | 100 *96* | 100 *73* | 50 *49* | 50 *37* | 25 |
| Tebuconazole 0.000025 ppm | 100 *94* | 88 *64* | | 25 *16* | 0 |
| Trifloxystrobin 0.0001 ppm | 100 *94* | | | | 0 |
| Pyraclostrobin 0.0001 ppm | 100 *94* | 67 *64* | | | 0 |
| Fluazinam 0.0063 ppm | 100 *96* | 100 *76* | 83 *55* | 67 *44* | 33 |
| Fluxapyroxad 0.000025 ppm | 100 *97* | 88 *82* | 75 *66* | | 50 |
| Prothioconazole 0.000025 ppm | 100 *97* | | | | 50 |
| Florylpicoxamid 0.0001 ppm | | | 50 *32* | 50 *16* | 0 |
| Benzovindiflupyr 0.000025 ppm | 100 *94* | 75 *64* | 90 *32* | | 0 |
| Mancozeb 0.0063 ppm | 100 *97* | | 75 *66* | | 50 |
| None | 94 | 64 | 32 | 16 | |

Each value in the table: (left) control value, (right italics) expected value calculated by Colby's equation.

[Table 14]

Result of soybean rust control test results (control value)-in the case where component (I) is Compound 8

| Component (II) \ Component (I): Compound 8 | 1.6 ppm | 0.4 ppm | 0.1 ppm | 0.025 ppm | 0.0063 ppm | None |
|---|---|---|---|---|---|---|
| Inpyrfluxam 0.00025 ppm | 100 *94* | 100 *79* | 75 *42* | | | 16 |
| Chlorothalonil 0.006 ppm | 100 *96* | 100 *86* | 75 *62* | 83 *53* | 58 *49* | 44 |
| Thiophanate-methyl 0.0001 ppm | 100 *95* | | 58 *50* | 58 *38* | | 27 |
| Tebuconazole 0.000025 ppm | 100 *96* | 100 *86* | 92 *62* | | | 44 |
| Trifloxystrobin 0.0001 ppm | 100 *96* | 92 *86* | 83 *62* | 83 *53* | 58 *49* | 16 |
| Fluazinam 0.006 ppm | 100 *6* | 100 *21* | 58 *16* | 49 *31* | 49 *25* | 16 |
| Prothioconazole 0.000025 ppm | 100 *96* | 100 *86* | 83 *62* | 58 *53* | | 44 |
| Florylpicoxamid 0.0001 ppm | 100 *94* | 100 *79* | | | | 27 |
| Metominostrobin 0.0004 ppm | 100 *95* | | | | | 27 |
| None | 93 | 75 | 32 | 16 | 10 | |

Each value in the table: (left) control value, (right italics) expected value calculated by Colby's equation.

[Table 15]

Result of soybean rust control test (control value)-in the case where component (I) is Compound 9

| Component (I): Compound 9 / Component (II) | 0.4 ppm | 0.1 ppm | 0.025 ppm | 0.0063 ppm | None |
|---|---|---|---|---|---|
| Azoxystrobin 0.0001 ppm | 100 *97* | 83 *66* | 67 *56* | | 33 |
| Inpyrfluxam 0.000025 ppm | 100 *98* | 100 *75* | | | 50 |
| Chlorothalonil 0.0063 ppm | 100 *97* | 83 *66* | 67 *56* | 67 *42* | 33 |
| Difenoconazole 0.000025 ppm | 100 *99* | 100 *87* | 75 *84* | | 75 |
| Cyproconazole 0.000025 ppm | 100 *97* | 67 *66* | 67 *56* | | 33 |
| Tebuconazole 0.000025 ppm | 100 *97* | 67 *66* | 67 *56* | | 33 |
| Trifloxystrobin 0.0001 ppm | 100 *96* | | | | 0 |
| Pyraclostrobin 0.0001 ppm | 100 *97* | 83 *66* | 67 *56* | | 33 |
| Fluazinam 0.0063 ppm | 100 *96* | 50 *49* | | | 0 |
| Fluxapyroxad 0.000025 ppm | 100 *96* | 100 *49* | | | 0 |
| Prothioconazole 0.000025 ppm | 100 *97* | 83 *66* | 67 *56* | 67 *42* | 33 |
| Florylpicoxamid 0.0001 ppm | 100 *96* | 100 *49* | 75 *34* | 50 *13* | 0 |
| Benzovindiflupyr 0.000025 ppm | 100 *98* | 100 *75* | | | 50 |
| Mancozeb 0.0063 ppm | 100 *96* | 50 *49* | | | 0 |
| None | 96 | 49 | 34 | 13 | |

Each value in the table: (left) control value, (right italics) expected value calculated by Colby's equation.

[Table 16]

Result of soybean rust control test (control value)-in the case where component (I) is Compound 10

| Component (I): Compound 10 / Component (II) | 1.6 ppm | 0.4 ppm | 0.1 ppm | 0.025 ppm | 0.0063 ppm | None |
|---|---|---|---|---|---|---|
| Inpyrfluxam 0.000025 ppm | | 83 *72* | 75 *51* | 66 *38* | | 16 |
| Chlorothalonil 0.006 ppm | 100 *97* | 92 *81* | 92 *67* | 83 *59* | 75 *44* | 44 |
| Thiophanate-methyl 0.0001 ppm | | 100 *75* | 83 *57* | 49 *47* | 41 *27* | 27 |
| Tebuconazole 0.000025 ppm | | 83 *81* | 100 *67* | 66 *59* | | 44 |
| Trifloxystrobin 0.0001 ppm | 100 *95* | 100 *72* | 66 *51* | 83 *38* | 33 *16* | 16 |
| Fluazinam 0.006 ppm | | 83 *72* | 66 *51* | 66 *38* | 58 *16* | 16 |
| Prothioconazole 0.000025 ppm | | | 75 *67* | 83 *59* | 75 *44* | 44 |
| Florylpicoxamid 0.0001 ppm | 100 *96* | | | 49 *47* | 33 *27* | 27 |
| Metominostrobin 0.0004 ppm | 100 *96* | 92 *75* | 83 *57* | 75 *47* | 66 *27* | 27 |
| None | 94 | 66 | 42 | 27 | 0 | |

Each value in the table: (left) control value, (right italics) expected value calculated by Colby's equation.

[Table 17]

Result of soybean rust control test (control value)-in the case where component (I) is Compound 11

| Component (II) \ Component (I): Compound 11 | | 1.6 ppm | 0.4 ppm | 0.1 ppm | 0.025 ppm | 0.0063 ppm | None |
|---|---|---|---|---|---|---|---|
| Inpyrfluxam | 0.000025 ppm | 100 *87* | | | 49 *28* | 33 *21* | 16 |
| Chlorothalonil | 0.02 ppm | | | | 83 *78* | | 75 |
| Thiophanate-methyl | 0.0004 ppm | 100 *95* | | | | | 66 |
| Tebuconazole | 0.0001 ppm | | 100 *96* | | 100 *88* | | 86 |
| Trifloxystrobin | 0.0001 ppm | 100 *87* | 92 *75* | | 66 *28* | 49 *21* | 16 |
| Fluazinam | 0.006 ppm | | | | 49 *28* | | 16 |
| Prothioconazole | 0.000025 ppm | 100 *91* | | 75 *73* | | | 44 |
| Florylpicoxamid | 0.0001 ppm | 100 *88* | 92 *78* | 92 *65* | 75 *38* | 33 *31* | 27 |
| Metominostrobin | 0.0004 ppm | 100 *88* | 83 *78* | | | 58 *31* | 27 |
| None | | 84 | 70 | 52 | 15 | 6 | |

Each value in the table: (left) control value, (right italics) expected value calculated by Colby's equation.

[Table 18]

Result of soybean rust control test (control value)-in the case where component (I) is Compound 12

| Component (II) \ Component (I): Compound 12 | | 0.4 ppm | 0.1 ppm | 0.025 ppm | 0.0063 ppm | None |
|---|---|---|---|---|---|---|
| Azoxystrobin | 0.0001 ppm | 100 *92* | | 50 *33* | | 0 |
| Inpyrfluxam | 0.000025 ppm | 100 *96* | | | | 50 |
| Chlorothalonil | 0.0063 ppm | 100 *92* | | | | 0 |
| Difenoconazole | 0.000025 ppm | 100 *93* | 100 *64* | | | 0 |
| Cyproconazole | 0.000025 ppm | 100 *92* | 100 *64* | 50 *33* | | 0 |
| Tebuconazole | 0.000025 ppm | 100 *94* | | 67 *55* | 67 *47* | 33 |
| Trifloxystrobin | 0.0001 ppm | 100 *98* | | | | 75 |
| Pyraclostrobin | 0.0001 ppm | 100 *92* | 100 *64* | 50 *33* | 50 *21* | 0 |
| Fluazinam | 0.0063 ppm | 100 *96* | | | | 50 |
| Fluxapyroxad | 0.000025 ppm | 100 *98* | 100 *91* | | | 75 |
| Prothioconazole | 0.000025 ppm | 100 *96* | | | | 50 |
| Florylpicoxamid | 0.0001 ppm | 100 *92* | | | | 0 |
| Benzovindiflupyr | 0.000025 ppm | 100 *96* | | | 75 *61* | 50 |
| Mancozeb | 0.0063 ppm | 100 *92* | | | | 0 |
| None | | 92 | 64 | 33 | 21 | |

Each value in the table: (left) control value, (right italics) expected value calculated by Colby's equation.

(Test Example) Fungicidal Test for Cucumber Anthracnose

The compounds were dissolved in dimethyl sulfoxide to prepare a chemical agent solution.

A mixture of the prescribed concentration of the chemical agent solution, cucumber anthracnose pathogen (*Colletotrichum lagenarium*) conidia, and Vogel medium was prepared on a microplate and incubated at 20°C in the dark for 7 days.

The turbidity at a wavelength of 405 nm was measured with a microplate reader, and the growth of cucumber anthracnose pathogen was compared with that of no treatment control to determine the growth inhibition rate. The results are shown in Tables 19 to 24, along with the expected values calculated as below.

Each expected value was calculated using Colby's equation:

$$E = M + N - (M \times N)/100$$

where E represents the expected value of the growth inhibition rate (%), M represents the growth inhibition rate (%) when the component (I) is used alone, and N represents the growth inhibition rate (%) when the component (II) is used alone.

In Tables 19 to 24, some favorable synergistic effect was observed for all combinations of components (I) and (II).

[Table 19]

Result of cucumber anthracnose fungicidal test (growth inhibition rate)-in the case where component (I) is Compound 3

| Component (II) \ Component (I): Compound 3 | | 0.4ppm | 0.1ppm | 0.025ppm | None |
|---|---|---|---|---|---|
| Ipflufenoquin | 0.000026ppm | 100 (84) | 100 (9) | 95 (26) | 0 |
| Iminoctadine trialbesilate | 0.0032ppm | | 93 (29) | 100 (42) | 22 |
| Cyflufenamid | 10ppm | 100 (94) | 84 (67) | 100 (73) | 64 |
| Cyprodinil | 0.00013ppm | 96 (86) | 72 (20) | 89 (35) | 12 |
| Potassium bicarbonate | 0.08ppm | 100 (92) | 79 (57) | 98 (65) | 53 |
| Thiophanate-methyl | 0.08ppm | 99 (84) | 95 (9) | 96 (26) | 0 |
| Picarbutrazox | 0.016ppm | | | 100 (90) | 87 |
| Fludioxonil | 0.0032ppm | | | 96 (73) | 64 |
| Florylpicoxamid | 0.0032ppm | 100 (84) | 69 (0) | 95 (26) | 0 |
| Metyltetraprole | 0.08ppm | 99 (96) | 98 (79) | 99 (83) | 77 |
| Mefentrifluconazole | 0.4ppm | 98 (94) | 86 (67) | 99 (73) | 64 |
| None | | 84 | 9 | 26 | |

Each value in the table: growth inhibition rate (%); (): expected value calculated by Colby's equation.

[Table 20]

Result of Cucumber anthracnose fungicidal test (growth inhibition rate)-in the case where component (I) is Compound 5

| Component (II) \ Component (I): Compound 5 | | 0.4ppm | 0.1ppm | 0.025ppm | None |
|---|---|---|---|---|---|
| Ipflufenoquin | 0.00064ppm | 97 (95) | 95 (87) | | 47 |
| Iminoctadine trialbesilate | 0.0032ppm | 99 (92) | 100 (81) | | 22 |
| Cyflufenamid | 10ppm | 100 (97) | 100 (91) | | 64 |
| Cyprodinil | 0.00013ppm | 100 (92) | 99 (79) | 62 (16) | 12 |
| Potassium bicarbonate | 0.08ppm | | 98 (89) | | 53 |
| Thiophanate-methyl | 0.4ppm | | | 71 (4) | 0 |
| Picarbutrazox | 0.016ppm | | | 99 (87) | 87 |
| Fludioxonil | 0.0032ppm | | 93 (91) | | 64 |
| Florylpicoxamid | 0.0032ppm | 100 (90) | 99 (76) | | 0 |
| Metyltetraprole | 0.08ppm | 99 (98) | 100 (94) | 100 (78) | 77 |
| Mefentrifluconazole | 0.4ppm | 99 (97) | 98 (91) | | 64 |
| None | | 90 | 76 | 4 | |

Each value in the table: growth inhibition rate (%); (): expected value calculated by Colby's equation.

[Table 21]

Result of cucumber anthracnose fungicidal test (growth inhibition rate)-in the case where component (I) is Compound 6

| Component (II) \ Component (I): Compound 6 | | 0.4ppm | 0.1ppm | 0.025ppm | None |
|---|---|---|---|---|---|
| Ipflufenoquin | 0.00064ppm | 97 (92) | 97 (82) | 95 (64) | 47 |
| Iminoctadine trialbesilate | 0.0032ppm | 99 (89) | | 69 (47) | 22 |
| Cyflufenamid | 10ppm | 100 (95) | 100 (88) | 79 (76) | 64 |
| Cyprodinil | 0.00013ppm | | 95 (71) | 97 (40) | 12 |
| Potassium bicarbonate | 0.08ppm | 100 (93) | 99 (85) | 100 (68) | 53 |
| Thiophanate-methyl | 0.4ppm | 100 (86) | | 44 (32) | 0 |
| Picarbutrazox | 0.00064ppm | 100 (86) | 83 (67) | | 0 |
| Fludioxonil | 0.0032ppm | | | 85 (76) | 64 |
| Florylpicoxamid | 0.0032ppm | 100 (86) | 97 (67) | 84 (32) | 0 |
| Metyltetraprole | 0.08ppm | 100 (97) | 100 (92) | 99 (85) | 77 |
| Mefentrifluconazole | 0.4ppm | 100 (95) | 100 (88) | 99 (76) | 64 |
| None | | 86 | 67 | 32 | |

Each value in the table: growth inhibition rate (%); (): expected value calculated by Colby's equation.

[Table 22]

Result of cucumber anthracnose fungicidal test (growth inhibition rate)-in the case where component (I) is Compound 8

| Component (II) \ Component (I): Compound 8 | | 0.4ppm | 0.1ppm | 0.025ppm | None |
|---|---|---|---|---|---|
| Ipflufenoquin | 0.00064ppm | 99 (92) | 80 (76) | | 47 |
| Iminoctadine trialbesilate | 0.0032ppm | | | 97 (67) | 22 |
| Cyflufenamid | 10ppm | 100 (95) | 100 (84) | 98 (85) | 64 |
| Cyprodinil | 0.00013ppm | | 88 (60) | 100 (63) | 12 |
| Potassium bicarbonate | 0.08ppm | 100 (93) | | 100 (81) | 53 |
| Thiophanate-methyl | 0.4ppm | 98 (85) | | | 0 |
| Picarbutrazox | 0.00064ppm | 100 (85) | 69 (55) | 100 (58) | 0 |
| Fludioxonil | 0.0032ppm | 97 (95) | 91 (84) | 100 (85) | 64 |
| Florylpicoxamid | 0.0032ppm | 98 (85) | 76 (55) | 74 (55) | 0 |
| Metyltetraprole | 0.08ppm | 99 (97) | 100 (90) | 99 (91) | 77 |
| Mefentrifluconazole | 0.4ppm | 98 (95) | 98 (84) | 99 (85) | 64 |
| None | | 85 | 55 | 58 | |

Each value in the table: growth inhibition rate (%); (): expected value calculated by Colby's equation.

[Table 23]

Result of cucumber anthracnose fungicidal test (growth inhibition rate)-in the case where component (I) is Compound 9

| Component (I): Compound 9 / Component (II) | | 0.4ppm | 0.1ppm | 0.025ppm | None |
|---|---|---|---|---|---|
| Ipflufenoquin | 0.00064ppm | 100 (91) | 98 (73) | 82 (69) | 47 |
| Iminoctadine trialbesilate | 0.0032ppm | 99 (87) | | 82 (55) | 22 |
| Cyflufenamid | 10ppm | 100 (94) | 100 (82) | 100 (79) | 64 |
| Cyprodinil | 0.000026ppm | | 88 (49) | | 0 |
| Potassium bicarbonate | 0.08ppm | 100 (92) | 80 (76) | | 53 |
| Thiophanate-methyl | 0.4ppm | 100 (83) | | 74 (42) | 0 |
| Picarbutrazox | 0.00064ppm | 100 (83) | 97 (49) | | 0 |
| Fludioxonil | 0.00064ppm | | 98 (49) | 87 (42) | 0 |
| Florylpicoxamid | 0.0032ppm | 100 (83) | 99 (49) | 65 (42) | 0 |
| Metyltetraprole | 0.08ppm | 99 (96) | 100 (88) | 97 (87) | 77 |
| Mefentrifluconazole | 0.4ppm | 98 (94) | 100 (82) | 99 (79) | 64 |
| None | | 83 | 49 | 42 | |

Each value in the table: growth inhibition rate (%); (): expected value calculated by Colby's equation.

[Table 24]

Result of cucumber anthracnose fungicidal test (growth inhibition rate)-in the case where component (I) is Compound 10

| Component (I): Compound 10 / Component (II) | | 0.4ppm | 0.1ppm | 0.025ppm | None |
|---|---|---|---|---|---|
| Ipflufenoquin | 0.00064ppm | 100 (98) | 99 (75) | 97 (80) | 47 |
| Iminoctadine trialbesilate | 0.0032ppm | | | 80 (71) | 22 |
| Cyflufenamid | 10ppm | 100 (99) | 100 (83) | 100 (87) | 64 |
| Cyprodinil | 0.00013ppm | 100 (96) | 99 (58) | | 12 |
| Potassium bicarbonate | 0.08ppm | 99 (98) | 100 (78) | | 53 |
| Thiophanate-methyl | 0.4ppm | 100 (96) | | 72 (63) | 0 |
| Picarbutrazox | 0.00064ppm | 100 (96) | 88 (52) | | 0 |
| Fludioxonil | 0.00013ppm | | | | 0 |
| Florylpicoxamid | 0.0032ppm | 99 (96) | 91 (52) | 97 (63) | 0 |
| Metyltetraprole | 0.08ppm | 100 (99) | 95 (89) | 99 (92) | 77 |
| Mefentrifluconazole | 0.4ppm | 100 (99) | 95 (83) | 96 (87) | 64 |
| None | | 96 | 52 | 63 | |

Each value in the table: growth inhibition rate (%); (): expected value calculated by Colby's equation.

The component (I) and component (II) may be used in combination to produce an agricultural/horticultural fungicidal composition with excellent plant disease control effects, which cannot be obtained using each single component alone.

When the component (I) is one of the above-mentioned compounds 1 to 12, for example, metarylpicoxamid other than the above-mentioned compounds may be used as the compound of component (II) to produce substantially the same effects.

## Industrial Applicability

Any of those selected randomly from the compositions of the present invention can exert the above effects.

From the above, it should be understood that any of the compositions of the present invention, including relevant compositions not shown in the Examples, is effective in controlling plant diseases while chemical damage is low and fungicidal effects are high, and is useful as an agricultural/horticultural fungicidal composition.

## Claims

1. An agricultural/horticultural fungicidal composition comprising component (I) and component (II) as active ingredients, wherein the component (I) is at least one compound selected from a compound of the formula (I):

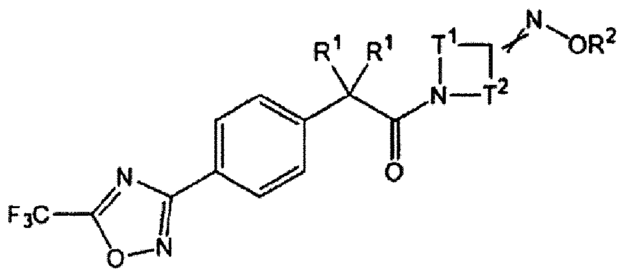

(I)

and a salt thereof, wherein

$R^1$ each independently represents a hydrogen atom, a $C_{1-6}$ alkyl group, or a $C_{1-6}$ alkoxy group;
$R^2$ represents a $C_{1-6}$ alkyl group, a $C_{1-6}$ alkyl group having at least one $G^1$, a $C_{2-6}$ chained unsaturated hydrocarbon group, a $C_{2-6}$ chained unsaturated hydrocarbon group having at least one $G^1$, a $C_{3-6}$ cycloalkyl group, a $C_{3-6}$ cycloalkyl group having at least one $G^2$, a $C_{6-10}$ aryl group, a $C_{6-10}$ aryl group having at least one $G^2$, a 5- to 6-membered heterocyclyl group, or a 5- to 6-membered heterocyclyl group having at least one $G^2$;
$G^1$ each independently represents a halogeno group, a hydroxyl group, a $C_{1-6}$ alkoxy group, a $C_{1-6}$ haloalkoxy group, a $C_{1-6}$ alkylthio group, a $C_{1-6}$ alkylsulfinyl group, a $C_{1-6}$ alkylsulfonyl group, a $C_{3-6}$ cycloalkyl group, a $C_{6-10}$ aryl group, a $C_{6-10}$ aryl group having at least one $g^1$, a 5- to 6-membered heterocyclyl group, a 5- to 6-membered heterocyclyl group having at least one $g^1$, or a cyano group;
$G^2$ each independently represents a halogeno group, a $C_{1-6}$ alkyl group, a $C_{1-6}$ haloalkyl group, a hydroxyl group, a $C_{1-6}$ alkoxy group, a $C_{1-6}$ alkylthio group, a $C_{1-6}$ alkylsulfinyl group, a $C_{1-6}$ alkylsulfonyl group, a $C_{3-6}$ cycloalkyl group, a $C_{6-10}$ aryl group, a $C_{6-10}$ aryl group having at least one $g^1$, a 5- to 6-membered heterocyclyl group, a 5- to 6-membered heterocyclyl group having at least one $g^1$, a nitro group, or a cyano group;
$g^1$ each independently represents a halogeno group, a $C_{1-6}$ alkyl group, a $C_{1-6}$ haloalkyl group, a $C_{1-6}$ alkoxy group, a $C_{1-6}$ haloalkoxy group, a nitro group, or a cyano group;
$T^1$ represents a $C_{1-2}$ alkylene group or a $C_{1-6}$ alkylene group having at least one $X^4$;
$T^2$ represents a $C_{1-2}$ alkylene group or a $C_{1-6}$ alkylene group having at least one $X^4$; and
$X^4$ each independently represents a halogeno group or a $C_{1-6}$ alkyl group, and

wherein the component (II) is a fungicide other than the component (I).

2. The agricultural/horticultural fungicidal composition according to claim 1, wherein the component (II) is at least one fungicide selected from the group consisting of the following:

(A) Agents acting on nucleic acid synthesis and metabolism:

A1) RNA polymerase I inhibitors
benalaxyl, benalaxyl-M, furalaxyl, metalaxyl, metalaxyl-M, oxadixyl, ofurace,
A2) adenosine deaminase inhibitors
bupirimate, dimethirimol, ethirimol,
A3) DNA/RNA biosynthesis inhibitors
hymexazole, octhilinone,
A4) DNA topoisomerase type II inhibitors
oxolinic acid,

(B) Agents acting on cytoskeleton and motor proteins:

B1) to B3) β-tubulin polymerization inhibitors
benomyl, carbendazim, fuberidazole, thiabendazole, thiophanate, thiophanate-methyl, diethofencarb, zoxamide, ethaboxam, chlorfenazole, debacarb, trichlamide, zarilamid,
B4) mitosis (point of action unknown) inhibitors pencycuron,
B5) delocalization inhibitors of spectrin-like proteins
fluopicolide, fluopimomide,
B6) actin/myosin/fimbrin function inhibitors
phenamacril, metrafenone, pyriofenone,

(C) agents acting on respiration:

C1) complex I:NADH oxidoreductase inhibitors
diflumetorim, tolfenpyrad, fenazaquin,
C2) complex II:succinate dehydrogenase inhibitors
benodanil, flutolanil, mepronil, isofetamid, fluopyram, cyclobutrifluram, fenfuram, carboxin, oxycarboxin, thifluzamide, benzovindiflupyr, bixafen, fluindapyr, fluxapyroxad, furametpyr, inpyrfluxam, isopyrazam, penflufen, penthiopyrad, sedaxane, isoflucypram, pydiflumetofen, boscalid, pyraziflumid, flubeneteram, furmecyclox,
C3) complex III:cytochrome bc1 (ubiquinol oxidase) Qo site (cyt b gene) inhibitors
azoxystrobin, coumoxystrobin, enoxastrobin, flufenoxystrobin, picoxystrobin, pyraoxystrobin, mandestrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, methyltetraprole,
C4) complex III:cytochrome bc1 (ubiquinone reductase) Qi site inhibitors
cyazofamid, amisulbrom, fenpicoxamid, florylpicoxamid, metarylpicoxamid,
C5) deconjugation inhibitors of oxidative phosphorylation binapacryl, dinocap, meptyldinocap, fluazinam,
C6) inhibitors of oxidative phosphorylation and ATP synthase fentin acetate, fentin chloride, fentin hydroxide,
C7) ATP transport inhibitors
silthiofam,
C8) complex III:cytochrome bc1 (ubiquinone reductase) Qo site, stigmatellin-binding subsite inhibitors
ametoctradin,

(D) agents acting on amino acid and protein synthesis:

D1) methionine biosynthesis (cgs gene) inhibitors
cyprodinil, mepanipyrim, pyrimethanil,
D2) protein synthesis (ribosomal translation termination step) inhibitors
blasticidin S
D3), D4) protein synthesis (ribosomal translation initiation step) inhibitors
kasugamycin, kasugamycin hydrochloride, streptomycin,
D5) protein synthesis (ribosomal polypeptide elongation step) inhibitors
oxytetracycline

(E) agents acting on signal transduction:

E1) signal transduction (mechanism of action unknown) inhibitors
quinoxyfen, proquinazid,
E2) MAP/histidine kinase (os-2, HOG1) inhibitors in osmotic signal transduction
fenpiclonil, fludioxonil,
E3) MAP/histidine kinase (os-1, Daf1) inhibitors in osmotic signal transduction
clozolinate, dimethachlone, iprodione, procymidone, vinclozolin,

(F) agents acting on lipid biosynthesis or transport/cell membrane structure or function:

F1) dicarboxyimide-based fungicides
F2) phospholipid biosynthesis, methyltransferase inhibitors edifenphos, iprobenfos, pyrazophos, isoprothiolane,
F3) cell peroxidation inhibitors

biphenyl, chloroneb, dicloran, quintozene, tecnazene, tolclofos-methyl, etridiazole,
F4) cell membrane permeability, fatty acid inhibitors iodocarb, propamocarb, propamocarb hydrochloride, prothiocarb,
F5) carboxylic acid amide (CAA)-based fungicides
F8) ergosterol binding inhibitors
natamycin,
F9) lipid homeostasis and transport/storage inhibitors oxathiapiprolin, fluoxapiprolin,

(G) inhibitors of sterol biosynthesis in cell membrane

G1) demethylase (erg11/cyp51) inhibitors at C14 position of sterol biosynthesis triforine, pyrifenox, pyrisoxazole, fenarimol, nuarimol, imazalil, oxpoconazole, pefurazoate, prochloraz, triflumizole, azaconazole, bitertanol, bromuconazole, cyproconazole, difenoconazole, diniconazole, epoxiconazole, etaconazole, fenbuconazole, fluquinconazole, flusilazole, flutriafol, hexaconazole, imibenconazole, ipconazole, mefentrifluconazole, metconazole, myclobutanil, penconazole, propiconazole, simeconazole, tebuconazole, tetraconazole, triadimefon, triadimenol, triticonazole, prothioconazole, fluoxytioconazole, furconazole, furconazole-cis, diniconazole-M,
G2) Δ14-reductase and Δ8→Δ7-isomerase (erg24, erg2) inhibitors in sterol biosynthesis
aldimorph, dodemorph, dodemorph acetate, fenpropimorph, tridemorph, fenpropidin, piperalin, spiroxamine, buthiobate,
G3) 3-keto reductase (erg27) inhibitors in C4-position demethylation of sterol biosynthetic system
fenhexamid, fenpyrazamine,
G4) squalene epoxidase (erg1) inhibitors of sterol biosynthesis system
pyributicarb, naftifine, terbinafine,

(H) cell wall biosynthesis inhibitors:

H4) chitin synthase inhibitors
polyoxin, polyoxorim,
H5) cellulose synthase inhibitors
dimethomorph, flumorph, pyrimorph, benthiavalicarb, iprovalicarb, valifenalate, mandipropamid,

(I) cell wall melanin synthesis inhibitors:

I1) reductase inhibitors in melanin biosynthesis
fthalide, pyroquilon, tricyclazole,
I2) dehydratase inhibitors in melanin biosynthesis
carpropamid, diclocymet, fenoxanil,
I3) polyketide synthase inhibitors in melanin biosynthesis tolprocarb,

(P) agents acting to induce resistance in host plants:

P01 to P03) agents involving salicylic acid signal transduction
acibenzolar-S-methyl, probenazole, tiadinil, isotianil,
P04) polysaccharide elicitors
laminarin,
P05) anthraquinone elicitors
extract from *Reynoutria sachalinensis*,
P06) microbial elicitors
cell walls of *Bacillus mycoides* isolate J, *Saccharomyces cerevisiae* strain LAS117,
P07) phosphonates
fosetyl-Al or phosphorous acids and salts thereof (including, potassium phosphite, calcium phosphite, aluminum phosphite, sodium phosphite),
P08) agents related to salicylic acid signal transduction dichlobentiazox,

(U) agents with unknown mechanism of action:
cymoxanil, teclofthalam, triazoxide, flusulfamide, diclomezine, cyflufenamid, dodine, dodine free base, flutianil, ferimzone, tebufloquin, picarbutrazox, validamycin, bethoxazin, cyprofuram, flumetover, nitrothalisopropyl, propamidine, ipflufenoquin, pyridachlometyl, pyrapropoyne, aminopyrifen, ipfentrifluconazole, quinofumelin, dipymetitrone, chloroinconazide, seboctylamine, flumetylsulforim, flufenoxadiazam,

(M) agents with multiple action point contact activity:

copper (different salts), basic copper sulfate, Bordeaux mixture, copper hydroxide, copper naphthenate, copper oxychloride, copper sulfate, cuprous oxide, oxine-copper, sulfur, lime sulfur, amobam, ferbam, mancozeb, maneb, metiram, propineb, thiram, zinc thiazole, zineb, ziram, captan, captafol, folpet, chlorothalonil, dichlofluanid, tolylfluanid; guazatine, guazatine acetates, iminoctadine, iminoctadine triacetate, iminoctadine trialbesilate, anilazine, dithianon, chinomethionat/quinomethionate, fluoroimide, methasulfocarb, dazomet, cufraneb, mancopper, polycarbamate,

(BM) biological pesticides/biological-derived pesticides with multiple mechanisms of action

a) plant extracts:

polypeptides (lectin), phenols, sesquiterpenes, triterpenoids, coumarins, terpene hydrocarbons, terpene alcohols, terpene phenols, extract from the cotyledons of lupine plantlets, extract from *Swinglea glutinosa,* extract from *Melaleuca alternifolia* (tea tree oil), plant oils (mixtures), eugenol, geraniol, thymol, alpha-pinene, alpha-terpinene, alpha-terpinol, alpha-terpinoline, gamma-terpinene, d-limonene, orange oil, linalool, menthol, ursolic acid, oleanolic acid, neem oil,

b) microorganisms (microbial strains or their extracts or metabolites):

fungi of the genus *Trichoderma,* including *Trichoderma atroviride, Trichoderma asperellum, Trichoderma harzianum, or Trichoderma virens,*

fungi of the genus *Gliocladium,* including *Gliocladium catenulatum,*

fungi of the genus *Clonostachys,* including *Cronostachys rosea,*

fungi of the genus *Coniothyrium,* including *Coniotylium minutans,*

fungi of the genus *Talaromyces,* incluidng *Talaromyces flavus,* yeast of the genus *Saccharomyces,* including *Saccharomyces cerevisiae,*

bacteria of the genus *Bacillus,* including *Bacillus amyloliquefaciens, Bacillus subtilis, Bacillus simplex,*

bacteria of the genus *Paenibacillus,*

bacteria of the genus *Burkholderia,*

fungi of the genus *Fusarium,*

bacteria of the genus *Pseudomonas,* including *Pseudomonas chlororaphis, Pseudomonas fluorescens, Pseudomonas rhodesiae,*

bacteria of the genus *Streptomyces,* including *Streptomyces griseovirides, Streptomyces lydicus,*

bacteria of the genus *Agrobacterium,* including *Agrobacterium radiobacter,*

bacteria of the genus *Erwinia,* including non-pathogenic *Erwinia carotovora,*

bacteria of the genus *Variovorax,* including *Variovorax paradoxus,*

bacteria of the genus *Lactobacillus,* including *Lactobacillus plantarum,*

c) other agents

possible elicitors, including yeast or its extract, β-glucan, chitin or chitosan or their fragments, β-aminobutyric acid, 2,6-dichloroisonicotinic acid, salicylic acid or its derivatives, algae extract, algae extract (hydrolysate), jasmine flower extract, sodium alginate, oligosaccharides, trehalose, polysaccharides, lipids, lipopolysaccharides, fatty acids, glycolipids, glycoproteins, glycopeptides, proteins or peptides derived from plants and/or pathogenic microorganisms, or ergosterol, and

(N) unclassified agents

mineral oils, organic oils, inorganic salts, material of natural origin, potassium bicarbonate, sodium hydrogen carbonate, calcium carbonate, calcium hydroxide, potassium iodide, potassium phosphonates, chitosan hydrochloride, and urea.

**3.** The agricultural/horticultural fungicidal composition according to claim 1 or 2, wherein the component (II) is at least one component selected from the group consisting of azoxystrobin, ipflufenoquin, impyrfluxam, epoxiconazole, chlorothalonil, cyazofamid, difenoconazole, cyproconazole, tebuconazole, triflumizole, trifloxystrobin, bixafen, picoxystrobin, pyraclostrobin, fluazinam, fluxapyroxad, prothioconazole, florylpicoxamid, benzovindiflupyr, mancozeb, iminoctadine trialbesilate, cyflufenamid, cyprodinil, potassium bicarbonate, thiophanate-methyl, picarbutrazox, fludioxonil, metyltetraprole, mefentrifluconazole, copper sulfate, and metominostrobin.

**4.** The agricultural/horticultural fungicidal composition according to any one of claims 1 to 3, wherein the component

(I) and the component (II) have a weight ratio of 1:1,000,000 to 1,000,000:1.

## INTERNATIONAL SEARCH REPORT

International application No. **PCT/JP2022/019639**

**A. CLASSIFICATION OF SUBJECT MATTER**

***A01N 43/836***(2006.01)i; ***A01N 37/34***(2006.01)i; ***A01N 37/50***(2006.01)i; ***A01N 43/40***(2006.01)i; ***A01N 43/42***(2006.01)i; ***A01N 43/50***(2006.01)i; ***A01N 43/54***(2006.01)i; ***A01N 43/56***(2006.01)i; ***A01N 43/653***(2006.01)i; ***A01N 47/14***(2006.01)i; ***A01N 47/24***(2006.01)i; ***A01N 55/02***(2006.01)i; ***A01P 3/00***(2006.01)i; ***C07D 413/10***(2006.01)i; ***C07D 413/14***(2006.01)i

FI: A01N43/836; A01P3/00; A01N43/54 A; A01N43/42 101; A01N43/56 C; A01N43/653 G; A01N37/34 104; A01N43/50 M; A01N43/653 C; A01N43/50 K; A01N37/50; A01N43/40 101E; A01N47/24 G; A01N43/40 101J; A01N47/14 C; A01N55/02 160; C07D413/10; C07D413/14

According to International Patent Classification (IPC) or to both national classification and IPC

**B. FIELDS SEARCHED**

Minimum documentation searched (classification system followed by classification symbols)

A01N43/836; A01N37/34; A01N37/50; A01N43/40; A01N43/42; A01N43/50; A01N43/54; A01N43/56; A01N43/653; A01N47/14; A01N47/24; A01N55/02; A01P3/00; C07D413/10; C07D413/14

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2019/022061 A1 (NIPPON SODA CO., LTD.) 31 January 2019 (2019-01-31) claims, paragraphs [0140]-[0161], [0182]-[0200], tables | 1-4 |
| A | JP 2018-536025 A (BASF SE) 06 December 2018 (2018-12-06) claims, paragraphs [0222]-[0327] | 1-4 |
| P, A | WO 2021/251274 A1 (NIPPON SODA CO., LTD.) 16 December 2021 (2021-12-16) claims, examples | 1-4 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 June 2022** | **12 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/019639**

| Patent document cited in search report | Publication date (day/month/year) | Patent family member(s) | Publication date (day/month/year) |
|---|---|---|---|
| WO 2019/022061 A1 | 31 January 2019 | EP 3660007 A1<br>claims, paragraphs [0110]-[0112], [0137]<br>KR 10-2020-0033256 A<br>CA 3069803 A<br>CN 111094258 A<br>BR 112020000992 A | |
| JP 2018-536025 A | 06 December 2018 | US 2018/0317491 A1<br>claims, paragraphs [0365]-[0475]<br>WO 2017/076742 A1<br>EP 3370524 A1<br>CA 3002299 A<br>KR 10-2018-0080286 A<br>CN 108347935 A<br>BR 112018008608 A | |
| WO 2021/251274 A1 | 16 December 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021080430 A
- WO 2019022061 A